# EUROPEAN PATENT SPECIFICATION

(11) **EP 4 502 595 B1**
(45) Date of publication and mention of the grant of the patent: **07.10.2026**
(21) Application number: 24189671.1
(22) Date of filing: 19.07.2024
(51) Int. Cl.: G01N 33/00

(54) **SYSTEM FOR NOTIFYING ENVIRONMENTAL POLLUTION STATUS**
SYSTEM ZUR MELDUNG DES UMWELTVERSCHMUTZUNGSSTATUS
SYSTÈME DE NOTIFICATION D'ÉTAT DE POLLUTION ENVIRONNEMENTALE

(30) Priority: 01.08.2023 US 202363530110 P; 26.09.2023 US 202363585277 P
(43) Date of publication of application: 05.02.2025
(73) Proprietor: Far Eastone Telecommunications Co., Ltd., Taipei City 10602 (TW)
(72) Inventor: YANG, Chia-Jui, 10602 Taipei City (TW); RAO, Herman Chunghwa, 10602 Taipei City (TW); KUO, Chun-Chieh, 10602 Taipei City (TW); CHIANG, Hua-Pei, 10602 Taipei City (TW); HSIAO, Shui-Shu, 10602 Taipei City (TW); CHANG, Zheng-Xiang, 10602 Taipei City (TW); JANG, Chyi-Dar, 10602 Taipei City (TW); WANG, Tsung-Jen, 10602 Taipei City (TW); LIAO, Che-Yu, 10602 Taipei City (TW); CHAN, Chih-Min, 10602 Taipei City (TW); YANG, Teng-Chieh, 10602 Taipei City (TW); HSU, Chang-Hung, 10602 Taipei City (TW)
(74) Representative: Balder IP Law, S.L.

(56) References cited:
- KR-A- 20220 167 786
- US-A1- 2014 320 648
- US-A1- 2020 064 322
- US-A1- 2021 222 903
- US-A1- 2021 255 113
- US-B2- 10 546 469
- US-B2- 9 332 322

## Description

### BACKGROUND OF THE INVENTION

This application claims priority for the US provisional patent application Nos. 63/530,110 filed on 1 August 2023, and 63/585,277 filed on 26 September 2023.

### Field of the Invention

The present invention relates to a notification system, particularly to a system for notifying environmental pollution status.

### Description of the Related Art

With the advancement of industry and commerce, modern people spend more and more time indoors. Most of the buildings in recent years have closed designs. Often due to poor ventilation in the indoor environment, air pollutants are easy to accumulate in the indoor environment, which affects human health. Compared with the outdoor environment, the concentration of indoor air pollutants is often higher than that of outdoor air pollutants. Therefore, the importance and safety of indoor air quality are more concerned and valued by people.

Generally, there are many sources of pollutants that cause indoor air pollution, such as cooking, smoking, spraying, or human activities of using indoor building materials, plywood, and resin adhesives, which may produce hazardous pollutants such as volatile organic compounds and suspended particles. Therefore, in addition to increasing the ventilation rate of the indoor environment, air purifiers can also be used to effectively remove indoor air pollutants in order to improve indoor air quality. However, air purifiers can only remove parts of the pollutants in the indoor space. No one knows which part of the indoor space has more pollutants and which part of the indoor space has fewer pollutants.

Document US10546469B2 describes a method that determines one or more sources of carbon monoxide (CO) in a smart-home environment that includes a plurality of smart devices that have at least measurement and communication capabilities. The method comprises measuring a level of CO in the smart-home environment to generate a CO measurement, and providing the CO measurement and one or more current characteristics of the smart-home environment, from one or more of the smart devices to an analyzing device. The method further comprises evaluating, by the analyzing device and with the CO measurement and the current characteristics of the smart-home environment, a set of CO correlation scenarios that attribute generation of CO to a corresponding one of a set of specific sources, and selecting one or more of the specific sources as the most likely source of the CO, by aggregating results of the correlation scenarios.

Document US2021222903A1 describes a sensor unit for personal health monitoring using smart home devices, which comprises an air quality sensor configured to generate air quality data that includes a value for volatile organic compounds. The sensor unit is configured to determine a first health metric indicative of a condition within a space in which the sensor unit is located based on the value in a first mode of operation, to determine a second health metric indicative of a person's health based on the value in a second mode of operation, and to generate a notification indicating at least one of the first health metric or the second health metric.

Document US2020064322A1 describes an electronic device for measuring gas, which comprises a sensor module and a processor for acquiring periphery state information indicating information on the outside of the electronic device and/or device state information indicating information on the inside of the electronic device, acquiring a measurement profile including information regarding target gas to be measured and a detection period of the target gas on the basis of the periphery state information and/or the device state information, and detecting the target gas through the sensor module according to the measurement profile.

Anyhow, in order to overcome the abovementioned problems, the present invention provides a system for notifying environmental pollution status, so as to solve the afore-mentioned problems of the prior art.

### SUMMARY OF THE INVENTION

The present invention provides a system for notifying environmental pollution status in accordance with independent claim 1, which implements environmental detection distribution in three-dimensional space to provide a recommended path. The system for notifying environmental pollution status includes a plurality of wireless environment sensing devices and a portable wireless environmental pollution status notification device. The wireless environment sensing devices are respectively arranged in different sensing locations of a physical environment and configured to respectively store the sensing locations and respectively sense pollution related information corresponding to the different sensing locations to output the pollution related information and the sensing locations corresponding thereto. The portable wireless environmental pollution status notification device is wirelessly connected to the plurality of wireless environment sensing devices, located in the physical environment, and configured to receive the pollution related information and the sensing locations corresponding thereto and generate notification signals based on the pollution related information and the sensing locations corresponding thereto.

In an embodiment of the present invention, the plurality of wireless environment sensing devices are wirelessly connected to the portable wireless environmental pollution status notification device using WiFi direct technology and Bluetooth (RTM) technology.

In accordance with the present invention, each of the plurality of wireless environment sensing devices includes at least one first pollutant sensor, a first image sensor, a first thermal imaging camera, a storage device, a first wireless communication module, and a first processor. The first pollutant sensor is configured to measure the first pollution parameter of first pollutants corresponding to the sensing location. The first image sensor and the first thermal imaging camera are configured to respectively capture a first visible light image and a first thermal image within the same range. The storage device is configured to store the sensing location. The first wireless communication module is wirelessly connected to the portable wireless environmental pollution status notification device. The first processor is electrically connected to the at least one first pollutant sensor, the first image sensor, the first thermal imaging camera, the storage device, and the first wireless communication module and configured to extract a first temperature range of the first thermal image, use the first pollution parameter as the pollution related information, and transmit the sensing location and the first pollution parameter to the portable wireless environmental pollution status notification device through the first wireless communication module.

In an embodiment of the present invention, the first pollution parameter is a pollution concentration, a wind speed, a pollution range, or a flame burning intensity.

In an embodiment of the present invention, when the first pollution parameter is greater than a given value, the first processor notifies the portable wireless environmental pollution status notification device through the first wireless communication module that the sensing location corresponding to the first pollution parameter greater than the given value is a polluted location.

In an embodiment of the present invention, when the first visible light image has a human face image and the first temperature range is greater than a given temperature for a given time period, the first processor notifies the portable wireless environmental pollution status notification device through the first wireless communication module that the sensing location corresponding to the first temperature range greater than the given temperature is a polluted location.

In an embodiment of the present invention, when the first visible light image has a human face image and the first temperature range is greater than a given temperature for a given time period, the first processor transmits the human face image to the portable wireless environmental pollution status notification device through the first wireless communication module.

In an embodiment of the present invention, when the first visible light image has a human face image and the first pollution parameter is greater than a given value for a given time period, the first processor transmits the human face image to the portable wireless environmental pollution status notification device through the first wireless communication module.

In an embodiment of the present invention, the first pollutant sensor is an optical finger navigation (OFN) sensor.

In an embodiment of the present invention, the first pollutants include bacteria, mold, viruses, flame, smoke, liquefied gas, carbon monoxide, carbon dioxide, ozone, alkane gases, benzene gases, ketone gases, natural gas, coal gas, gasoline, chlorine, ammonia, flammable gases, harmful volatile matter, or airborne matter.

In accordance with the present invention, the notification signals include an image signal, a sound signal, and a vibration signal. The portable wireless environmental pollution status notification device includes a second wireless communication module, a second processor, a portable display, at least one portable sound player, and a portable vibrator. The second wireless communication module is wirelessly connected to the first wireless communication module. The second processor is electrically connected to the second wireless communication module and configured to receive the sensing location and the first pollution parameter through the second wireless communication module. The portable display is electrically connected to the second processor. The second processor is configured to drive the portable display to generate the image signal based on a given condition, the sensing location, and the first pollution parameter. The portable sound player is electrically connected to the second processor. The second processor is configured to drive the at least one portable sound player to generate the sound signal based on the sensing location and the first pollution parameter. The portable vibrator is electrically connected to the second processor. The second processor is configured to drive the portable vibrator to generate the vibration signal based on the sensing location and the first pollution parameter.

In an embodiment of the present invention, the at least one portable sound player includes a plurality of portable sound players.

In accordance with the present invention, the given condition includes one of the different sensing locations as an ending location. The second processor is configured to receive reference signal received power (RSRP), signal-to-interference-plus-noise ratios (SINRs), packet round-trip time (RTT), angles of arrival, and angles of departure from the first wireless communication module through the second wireless communication module and calculate a starting location based on at least three of the different sensing locations and the RSRP, the SINRs, the packet RTT, the angles of arrival, and the angles of departure corresponding thereto. The second processor is configured to plan candidate paths connected between the ending location and the starting location based on the different sensing locations, calculate risk values corresponding to the candidate paths, and select the candidate path corresponding to the highest one or the lowest one of the risk values as a recommended path. The risk $value = {\sum }_{i = 1}^{n} \frac{Pi}{Di}$*.* Di represents a distance between the starting location and the i-th sensing location corresponding to the candidate path. Pi represents the first pollution parameter measured in the i-th sensing location corresponding to the candidate path. n represents the total number of the sensing locations corresponding to the candidate path. The image signal shows the recommended path and an image corresponding to the sensing locations, the first pollutants, and the first pollution parameter.

In an embodiment of the present invention, the portable wireless environmental pollution status notification device further includes at least one second pollutant sensor, a second image sensor, and a second thermal imaging camera. The second pollutant sensor is electrically connected to the second processor and configured to measure a second pollution parameter of second pollutants corresponding to the starting location. The second pollutants and the first pollutants belong to identical pollutants and the second pollution parameter and the first pollution parameter belong to identical pollution parameters. The second image sensor and the second thermal imaging camera are electrically connected to the second processor and configured to respectively capture a second visible light image and a second thermal image within the same range corresponding to the starting location. The second processor is configured to obtain the second temperature range of the second thermal image. When the sensing location is the starting location, the second processor respectively uses the second pollution parameter and the second temperature range to replace the corresponding first pollution parameter and the corresponding first temperature range.

In an embodiment of the present invention, when the second pollution parameter is greater than a given value, the second processor drives the portable display to display that the sensing location corresponding to the second pollution parameter greater than the given value is a polluted location.

In an embodiment of the present invention, when the second visible light image has a human face image and the second temperature range is greater than a given temperature for a given time period, the second processor drives the portable display to display the sensing position corresponding to the second temperature greater than the given temperature is a polluted location.

In an embodiment of the present invention, when the second visible light image has a human face image and the second temperature range is greater than a given temperature, the second processor drives the portable display to display the human face image as a source of infection.

In an embodiment of the present invention, when the second visible light image has a human face image and the second pollution parameter is greater than a given value for a given time period, the second processor drives the portable display to display the human face image as a source of infection.

In accordance with the present invention, a system for notifying environmental pollution status in accordance with independent system claim 16 further includes at least one wireless base station and a cloud server. The wireless base station is wirelessly connected to the plurality of wireless environment sensing devices and the portable wireless environmental pollution status notification device. The cloud server is electrically connected to the wireless base station. The plurality of wireless environment sensing devices are configured to transmit the pollution related information and the sensing locations corresponding thereto to the cloud server through the wireless base station. The cloud server is configured to transmit the pollution related information and the sensing locations corresponding thereto to the portable wireless environmental pollution status notification device through the wireless base station.

In an embodiment of the present invention, the wireless base station is a 3G base station, a 4G base station, a 5G base station, a Bluetooth (RTM) base station, or a WiFi base station.

In an embodiment of the present invention, each of the plurality of wireless environment sensing devices includes at least one first pollutant sensor, a first image sensor, a first thermal imaging camera, a storage device, a first wireless communication module, and a first processor. The first pollutant sensor is configured to measure the first pollution parameter of first pollutants corresponding to the sensing location. The first image sensor and the first thermal imaging camera are configured to respectively capture a first visible light image and a first thermal image within the same range. The storage device is configured to store the sensing location. The first wireless communication module is wirelessly connected to the wireless base station. The first processor is electrically connected to the first pollutant sensor, the first image sensor, the first thermal imaging camera, the storage device, and the first wireless communication module and configured to extract the first temperature range of the first thermal image, use the first pollution parameter as the pollution related information, and transmit the sensing location and the first pollution parameter to the cloud server through the first wireless communication module and the wireless base station.

In an embodiment of the present invention, the first pollution parameter is a pollution concentration, a wind speed, a pollution range, or a flame burning intensity.

In an embodiment of the present invention, when the first pollution parameter is greater than a given value, the first processor notifies the portable wireless environmental pollution status notification device through the first wireless communication module, the wireless base station, and the cloud server that the sensing location corresponding to the first pollution parameter greater than the given value is a polluted location.

In an embodiment of the present invention, when the first visible light image has a human face image and the first temperature range is greater than a given temperature for a given time period, the first processor notifies the portable wireless environmental pollution status notification device through the first wireless communication module, the wireless base station, and the cloud server that the sensing location corresponding to the first temperature range greater than the given temperature is a polluted location.

In an embodiment of the present invention, when the first visible light image has a human face image and the first temperature range is greater than a given temperature for a given time period, the first processor transmits the human face image to the portable wireless environmental pollution status notification device through the first wireless communication module, the wireless base station, and the cloud server.

In an embodiment of the present invention, when the first visible light image has a human face image and the first pollution parameter is greater than a given value for a given time period, the first processor transmits the human face image to the portable wireless environmental pollution status notification device through the first wireless communication module, the wireless base station, and the cloud server.

In an embodiment of the present invention, the first pollutant sensor is an optical finger navigation (OFN) sensor.

In an embodiment of the present invention, the first pollutants include bacteria, mold, viruses, flame, smoke, liquefied gas, carbon monoxide, carbon dioxide, ozone, alkane gases, benzene gases, ketone gases, natural gas, coal gas, gasoline, chlorine, ammonia, flammable gases, harmful volatile matter, or airborne matter.

In an embodiment of the present invention, the notification signals include an image signal, a sound signal, and a vibration signal. The portable wireless environmental pollution status notification device includes a second wireless communication module, a second processor, a portable display, at least one portable sound player, and a portable vibrator. The second wireless communication module is wirelessly connected to the wireless base station. The second processor is electrically connected to the second wireless communication module and configured to receive the sensing location and the first pollution parameter through the second wireless communication module. The portable display is electrically connected to the second processor. The second processor is configured to drive the portable display to generate the image signal based on a given condition, the sensing location, and the first pollution parameter. The portable sound player is electrically connected to the second processor. The second processor is configured to drive the portable sound player to generate the sound signal based on the sensing location and the first pollution parameter. The portable vibrator is electrically connected to the second processor. The second processor is configured to drive the portable vibrator to generate the vibration signal based on the sensing location and the first pollution parameter.

In an embodiment of the present invention, the at least one portable sound player includes a plurality of portable sound players.

In accordance with the present invention, the given condition includes one of the different sensing locations as an ending location. The second processor is configured to receive reference signal received power (RSRP), signal-to-interference-plus-noise ratios (SINRs), packet round-trip time (RTT), angles of arrival, angles of departure, and the location of the wireless base station from the wireless base station through the second wireless communication module and calculate a starting location based on the RSRP, the SINRs, the packet RTT, the angles of arrival, the angles of departure, and the location of the wireless base station. The second processor is configured to plan candidate paths connected between the ending location and the starting location based on the different sensing locations, calculate risk values corresponding to the candidate paths, and select the candidate path corresponding to the highest one or the lowest one of the risk values as a recommended path. The risk $value = {\sum }_{i = 1}^{n} \frac{Pi}{Di}$*,* Di represents a distance between the starting location and the i-th sensing location corresponding to the candidate path. Pi represents the first pollution parameter measured in the i-th sensing location corresponding to the candidate path. n represents the total number of the sensing locations corresponding to the candidate path. The image signal shows the recommended path and an image corresponding to the sensing locations, the first pollutants, and the first pollution parameter.

In an embodiment of the present invention, the portable wireless environmental pollution status notification device further includes at least one second pollutant sensor, a second image sensor and a second thermal imaging camera. The second pollutant sensor is electrically connected to the second processor and configured to measure the second pollution parameter of second pollutants corresponding to the starting location. The second pollutants and the first pollutants belong to identical pollutants and the second pollution parameter and the first pollution parameter belong to identical pollution parameters. The second image sensor and the second thermal imaging camera are electrically connected to the second processor and configured to respectively capture a second visible light image and a second thermal image within the same range corresponding to the starting location. The second processor is configured to obtain the second temperature range of the second thermal image. When the sensing location is the starting location, the second processor respectively uses the second pollution parameter and the second temperature range to replace the corresponding first pollution parameter and the corresponding first temperature range.

In an embodiment of the present invention, when the second pollution parameter is greater than a given value, the second processor drives the portable display to display that the sensing location corresponding to the second pollution parameter greater than the given value is a polluted location.

In an embodiment of the present invention, when the second visible light image has a human face image and the second temperature range is greater than a given temperature for a given time period, the second processor drives the portable display to display the sensing position corresponding to the second temperature greater than the given temperature is a polluted location.

In an embodiment of the present invention, when the second visible light image has a human face image and the second temperature range is greater than a given temperature, the second processor drives the portable display to display the human face image as a source of infection.

In an embodiment of the present invention, when the second visible light image has a human face image and the second pollution parameter is greater than a given value for a given time period, the second processor drives the portable display to display the human face image as a source of infection.

To sum up, the system for notifying environmental pollution status uses the wireless environment sensing devices to implement environmental detection distribution in three-dimensional space, thereby providing a recommended path.

Below, the embodiments are described in detail in cooperation with the drawings to make easily understood the technical contents, characteristics and accomplishments of the present invention.

### BRIEF DESCRIPTION OF THE DRAWINGS

- Fig.1: is a schematic diagram illustrating a system for notifying environmental pollution status according to an embodiment of the present invention;
- Fig.2: is a schematic diagram illustrating a wireless environment sensing device according to an embodiment of the present invention;
- Fig.3: is a schematic diagram illustrating a first pollutant sensor according to an embodiment of the present invention;
- Fig.4: is a schematic diagram illustrating a portable wireless environmental pollution status notification device according to an embodiment of the present invention;
- Fig.5: is a schematic diagram illustrating sensing locations according to an embodiment of the present invention; and
- Fig.6: is a schematic diagram illustrating an image displayed by a portable display according to an embodiment of the present invention.

### DETAILED DESCRIPTION OF THE INVENTION

Reference will now be made in detail to embodiments illustrated in the accompanying drawings. Wherever possible, the same reference numbers are used in the drawings and the description to refer to the same or like parts. In the drawings, the shape and thickness may be exaggerated for clarity and convenience. This description will be directed in particular to elements forming part of, or cooperating more directly with, methods and apparatus in accordance with the present disclosure. It is to be understood that elements not specifically shown or described may take various forms well known to those skilled in the art. Many alternatives and modifications will be apparent to those skilled in the art, once informed by the present disclosure.

When an element is referred to as being "on" another element, it can be directly on the other element or intervening elements may be present therebetween. In contrast, when an element is referred to as being "directly on" another element, there are no intervening elements present. As used herein, the term "and/or" includes any and all combinations of one or more of the associated listed items.

Reference throughout this specification to "one embodiment" or "an embodiment" means that a particular feature, structure, or characteristic described in connection with the embodiment is included in at least one embodiment. Thus, the appearances of the phrases "in one embodiment" or "in an embodiment" in various places throughout this specification are not necessarily all referring to the same embodiment.

The invention is particularly described with the following examples which are only for instance. Those skilled in the art will readily observe that numerous modifications and alterations of the device and method may be made while retaining the teachings of the invention. Accordingly, the following disclosure should be construed as limited only by the metes and bounds of the appended claims. In the whole patent application and the claims, except for clearly described content, the meaning of the articles "a" and "the" includes the meaning of "one or at least one" of the elements or components. Moreover, in the whole patent application and the claims, except that the plurality can be excluded obviously according to the context, the singular articles also contain the description for the plurality of elements or components. In the entire specification and claims, unless the contents clearly specify the meaning of some terms, the meaning of the article "wherein" includes the meaning of the articles "wherein" and "whereon". The meanings of every term used in the present claims and specification refer to a usual meaning known to one skilled in the art unless the meaning is additionally annotated. Some terms used to describe the invention will be discussed to guide practitioners about the invention. The examples in the present specification do not limit the claimed scope of the invention.

Furthermore, it can be understood that the terms "comprising," "including," "having," "containing," and "involving" are open-ended terms, which refer to "may include but is not limited to so." In addition, each of the embodiments or claims of the present invention is not necessary to achieve all the effects and advantages possibly to be generated, and the abstract and title of the present invention is used to assist for patent search and is not used to further limit the claimed scope of the present invention.

Further, in the present specification and claims, the term "comprising" is open type and should not be viewed as the term "consisted of." In addition, the term "electrically coupled" can be referring to either directly connecting or indirectly connecting between elements. Thus, if it is described in the below contents of the present invention that a first device is electrically coupled to a second device, the first device can be directly connected to the second device, or indirectly connected to the second device through other devices or means. Moreover, when the transmissions or generations of electrical signals are mentioned, one skilled in the art should understand some degradations or undesirable transformations could be generated during the operations. If it is not specified in the specification, an electrical signal at the transmitting end should be viewed as substantially the same signal as that at the receiving end. For example, when the end A of an electrical circuit provides an electrical signal S to the end B of the electrical circuit, the voltage of the electrical signal S may drop due to passing through the source and drain of a transistor or due to some parasitic capacitance. However, the transistor is not deliberately used to generate the effect of degrading the signal to achieve some result, that is, the signal S at the end A should be viewed as substantially the same as that at the end B.

Unless otherwise specified, some conditional sentences or words, such as "can", "could", "might", or "may", usually attempt to express what the embodiment in the present invention has, but it can also be interpreted as a feature, element, or step that may not be needed. In other embodiments, these features, elements, or steps may not be required.

In the following description, a system for notifying environmental pollution status will be provided, which uses wireless environment sensing devices to implement environmental detection distribution in three-dimensional space, thereby providing a recommended path.

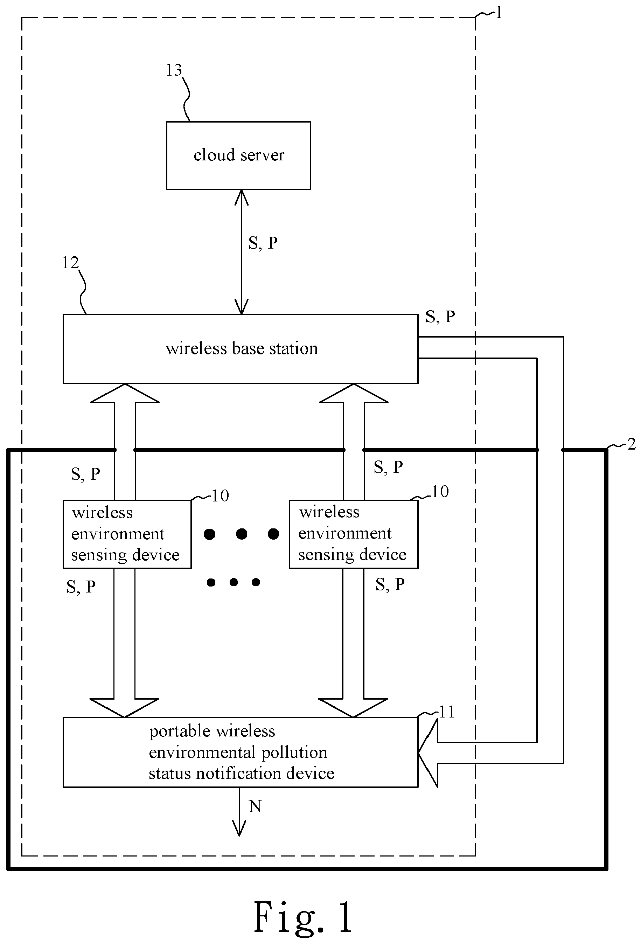

Fig.1 is a schematic diagram illustrating a system for notifying environmental pollution status according to an embodiment of the present invention. Referring to Fig.1, a system 1 for notifying environmental pollution status is introduced as follows. The system 1 for notifying environmental pollution status includes a plurality of wireless environment sensing devices 10 and a portable wireless environmental pollution status notification device 11. The portable wireless environmental pollution status notification device 11 may be installed on a user and follows the user's movements. The wireless environment sensing devices 10 are respectively arranged in the different sensing locations S of a physical environment 2. The portable wireless environmental pollution status notification device 11 is wirelessly connected to the wireless environment sensing devices 10 and located in the physical environment 2. For example, the wireless environment sensing devices 10 are wirelessly connected to the portable wireless environmental pollution status notification device 11 using WiFi direct technology and Bluetooth (RTM) technology. The wireless environment sensing devices 10 respectively store the sensing locations S and respectively sense pollution related information P corresponding to the different sensing locations S to output the pollution related information P and the sensing locations S corresponding thereto. The portable wireless environmental pollution status notification device 11 receives the pollution related information P and the sensing locations S corresponding thereto and generates notification signals N based on the pollution related information P and the sensing locations S corresponding thereto. The notification signals N may include an image signal, a sound signal, and a vibration signal. The sensing location S can be expressed in a rectangular coordinate system or a spherical coordinate system.

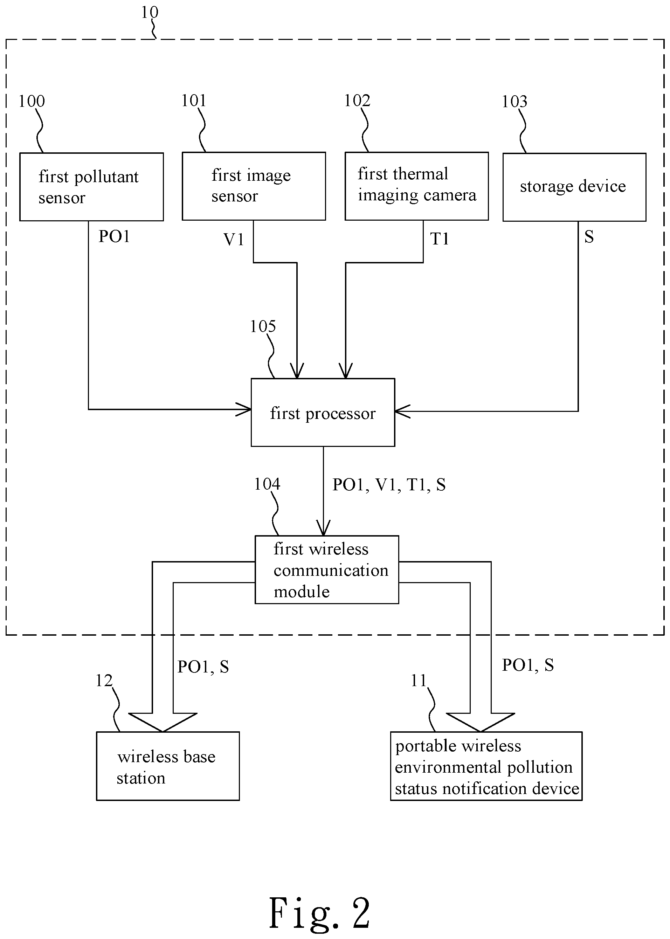

Fig.2 is a schematic diagram illustrating a wireless environment sensing device according to an embodiment of the present invention. Referring to Fig.2, each of the wireless environment sensing devices 10 includes at least one first pollutant sensor 100, a first image sensor 101, a first thermal imaging camera 102, a storage device 103, a first wireless communication module 104, and a first processor 105. For convenience and clarity, the number of the first pollutant sensor 100 is one. The first wireless communication module 104 is wirelessly connected to the portable wireless environmental pollution status notification device **11** using WiFi direct technology and Bluetooth (RTM) technology. The first processor 105 is electrically connected to the first pollutant sensor 100, the first image sensor 101, the first thermal imaging camera 102, the storage device 103, and the first wireless communication module 104. The first pollutant sensor 100 measures the first pollution parameter PO1 of first pollutants corresponding to the sensing location S. For example, the first pollutants may include, but are not limited to, bacteria, mold, viruses, flame, smoke, liquefied gas, carbon monoxide, carbon dioxide, ozone, alkane gases, benzene gases, ketone gases, natural gas, coal gas, gasoline, chlorine, ammonia, flammable gases, harmful volatile matter, or airborne matter. The first pollutant sensor 100 may be, but not limited to, an optical finger navigation (OFN) sensor, a wind speed sensor, a chemical sensor, or an optical sensor. When the pollutant is carbon monoxide, the carbon monoxide will absorb the infrared rays with a wavelength of about 4700 nanometers generated by the OFN sensor. The carbon monoxide will reflect the infrared rays back to the OFN sensor. The difference between the intensities of the reflected and generated infrared rays is used to calculate the concentration of carbon monoxide. The first pollution parameter PO1 may be, but not limited to, a pollution concentration, a wind speed, a pollution range, or a flame burning intensity. The first image sensor 101 and the first thermal imaging camera 102 respectively capture a first visible light image V1 and a first thermal image T1 within the same range. The storage device 103 stores the sensing location S. The first processor 105 extracts the first temperature range of the first thermal image T1, uses the first pollution parameter PO1 as the pollution related information, and transmits the sensing location S and the first pollution parameter PO1 to the portable wireless environmental pollution status notification device **11** through the first wireless communication module 104.

When the first pollution parameter PO1 is greater than a given value, the first processor 105 notifies the portable wireless environmental pollution status notification device 11 through the first wireless communication module 104 that the sensing location S corresponding to the first pollution parameter PO1 greater than the given value is a polluted location. When the first visible light image V1 has a human face image and the first temperature range is greater than a given temperature for a given time period, the first processor 105 notifies the portable wireless environmental pollution status notification device 11 through the first wireless communication module 104 that the sensing location S corresponding to the first temperature range greater than the given temperature is a polluted location. When the first visible light image V1 has a human face image and the first temperature range is greater than a given temperature for a given time period, the first processor 105 transmits the human face image to the portable wireless environmental pollution status notification device 11 through the first wireless communication module 104. When the first visible light image V1 has a human face image and the first pollution parameter PO1 is greater than a given value for a given time period, the first processor 105 transmits the human face image to the portable wireless environmental pollution status notification device 11 through the first wireless communication module 104.

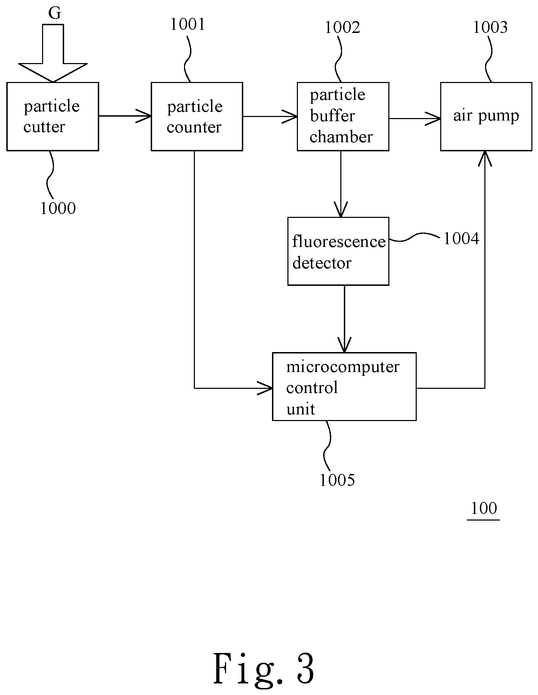

Fig.3 is a schematic diagram illustrating a first pollutant sensor according to an embodiment of the present invention. Please refer to Fig.2 and Fig.3. When the first pollutant is a bioaerosol containing bacteria, mold, or viruses, the first pollutant sensor 100 may include a particle cutter 1000, a particle counter 1001, a particle buffer chamber 1002, an air pump 1003, a fluorescence detector 1004, and a microcomputer control unit 1005. The particle cutter 1000, the particle counter 1001, the particle buffer chamber 1002, and the air pump 1003 are connected in sequence through gas pipelines. The particle buffer chamber 1002 is connected to the fluorescence detector 1004 through a gas pipeline. The microcomputer control unit 1005 is electrically connected to the first processor 105, the particle counter 1001, and the air pump 1003. When the air pump 1003 starts to operate, the air pump 1003 absorbs a bioaerosol G and transmits it to the particle cutter 1000. The particle cutter 1000 can filter out particles, water droplets, oil droplets and smoke particles with a particle diameter greater than 10 microns (µm) in the sampled bioaerosol G to avoid interference caused by non-biological aerosols that can produce fluorescence, thereby reducing the error rate of the device. The particle counter 1001 may operate based on the light scattering principle. The particle counter 1001 is placed before the particle buffer chamber 1002 and after the particle cutter 1000 to determine the optical equivalent particle diameter and the concentration of the particles of the bioaerosol G to be measured. The fluorescence detector 1002 is used to detect the fluorescence intensity of the particle clusters of the bioaerosol G in the particle buffer chamber 1002. Specifically, the fluorescence detector 1002 includes an excitation light source and a photodetector. The photodetector is used to obtain the fluorescence intensity of the particle cluster of the bioaerosol G excited by the excitation light in the particle buffer chamber 1002, convert the fluorescence intensity into an electrical signal, and transmit the electrical signal to the microcomputer control unit 1005. The microcomputer control unit 1005 divides the fluorescence intensity by the atmospheric volume to obtain the concentration of the bioaerosol G and uses the concentration of the bioaerosol G as the concentration of pollutants. The first pollutant sensor 100 can also refer to Chinese patent CN103091291A.

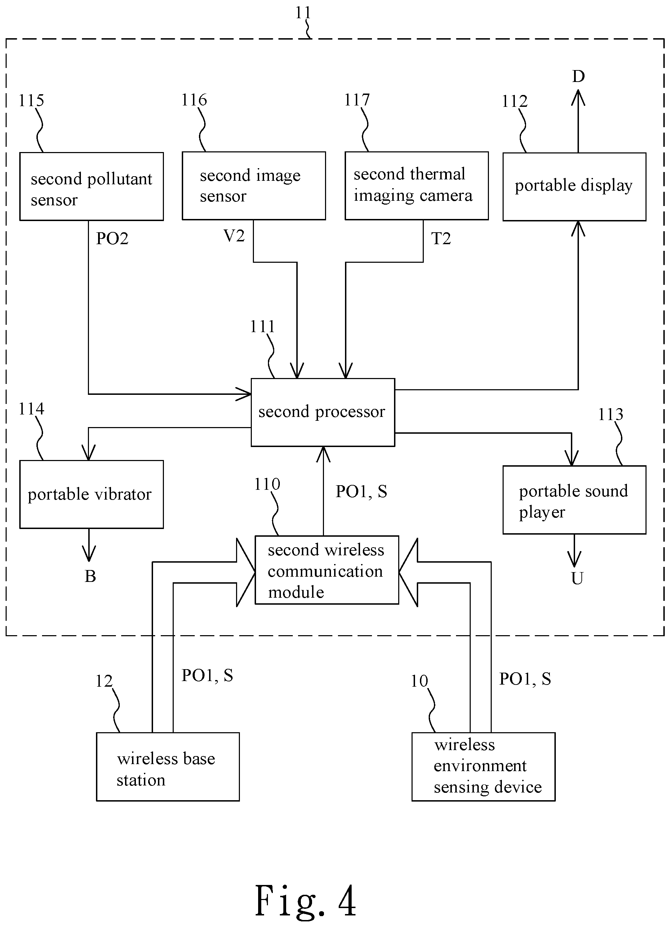

Fig.4 is a schematic diagram illustrating a portable wireless environmental pollution status notification device according to an embodiment of the present invention. Referring to Fig.4 and Fig.2, the portable wireless environmental pollution status notification device **11** may include a second wireless communication module 110, a second processor **111,** a portable display 112, at least one portable sound player 113, and a portable vibrator 114. For convenience and clarity, the number of the portable sound player 113 is one. The second processor **111 is** electrically connected to the second wireless communication module 110, the portable display 112, the portable sound player 113, and the portable vibrator 114. The second wireless communication module 110 is wirelessly connected to the first wireless communication module 104 using WiFi direct technology and Bluetooth (RTM) technology. The second processor **111** receives the sensing location S and the first pollution parameter PO1 through the second wireless communication module 110. The second processor **111** drives the portable display 112 to generate the image signal D based on a given condition, the sensing location S, and the first pollution parameter PO1. The second processor 111 may drive the portable display 112 to display the detected human face image or the detected polluted location and notify the user that the human face image or the polluted location is a potential source of pollution. The second processor 111 may drive the portable sound player 113 to generate the sound signal U based on the sensing location S and the first pollution parameter PO1. For example, different first pollution parameters PO1 respectively correspond to different sound signals U. If there are a plurality of portable sound players 113, the portable sound players 113 are respectively arranged on different sides of the user. The second processor 111 drives the portable vibrator 114 to generate the vibration signal B based on the sensing location S and the first pollution parameter PO1. For example, different first pollution parameters PO1 respectively correspond to different vibration frequencies or different vibration intensities of the vibration signals B.

The given condition includes one of the different sensing locations S as an ending location. The second processor 111 receives reference signal received power (RSRP), signal-to-interference-plus-noise ratios (SINRs), packet round-trip time (RTT), angles of arrival, and angles of departure from the first wireless communication module 104 through the second wireless communication module 110 and calculates the location of the second processor 111 as a starting location based on at least three of the different sensing locations S and the RSRP, the SINRs, the packet RTT, the angles of arrival, and the angles of departure corresponding thereto. The starting location is known. Thus, when the first pollution parameter PO1 corresponding to the starting location close to the sensing location S is greater, the intensity of the corresponding sound signal U or vibration signal B is greater or the vibration frequency of the corresponding vibration signal B is greater.

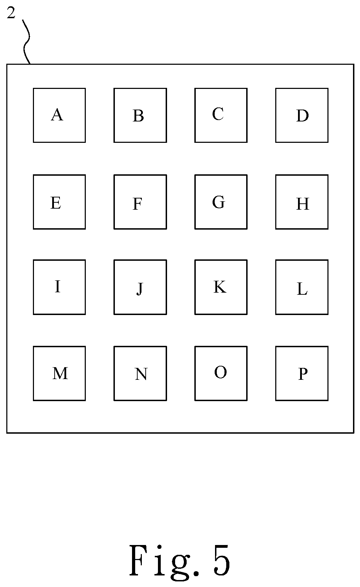

The second processor 111 plans candidate paths connected between the ending location and the starting location based on the different sensing locations S, calculates risk values corresponding to the candidate paths, and selects the candidate path corresponding to the highest one or the lowest one of the risk values as a recommended path. The risk $value = {\sum }_{i = 1}^{n} \frac{Pi}{Di}$ *.* Di represents a distance between the starting location and the i-th sensing location corresponding to the candidate path. Pi represents the first pollution parameter PO1 measured in the i-th sensing location S corresponding to the candidate path. n represents the total number of the sensing locations S corresponding to the candidate path. The image signal D shows the recommended path and an image corresponding to the sensing locations S, the first pollutants, and the first pollution parameter PO1. Fig.5 is a schematic diagram illustrating sensing locations according to an embodiment of the present invention. Refer to Fig.4 and Fig.5. Assume that the starting location is the location of area M and that the ending location is the location of area A. There are two candidate paths in total. The first candidate path includes areas I, E, and A and the second candidate path includes areas N, J, F, B, and A. Assume that coefficients Di corresponding to areas I, N, E, J, F, B and A are 1, 1, 2, 1.5, 2.5, 3.5 and 3 respectively. The first pollution parameters PO1 corresponding to areas I, N, E, J, F, B and A are respectively 5, 1, 2, 3, 8, 1 and 6. Therefore, the risk value of the first candidate path =1/1+2/2+6/3=4. The risk value of the second candidate path=1/1+3/1.5+8/2.5+1/3.5+6/3=8.2+2/7. In other words, the risk value of the second candidate path is greater than the risk value of the first candidate path. If one intends to avoid pollution sources, the first candidate path is selected as the recommended path. If one intends to clean up the pollution source, the second candidate path is selected as the recommended path.

Referring to Fig.4, the portable wireless environmental pollution status notification device 11 may further include at least one second pollutant sensor 115, a second image sensor 116, and a second thermal imaging camera 117. For convenience and clarity, the number of the second pollutant sensor 115 is one. The second pollutant sensor 115, the second image sensor 116, and the second thermal imaging camera 117 are electrically connected to the second processor 111. The second pollutant sensor 115 measures the second pollution parameter PO2 of second pollutants corresponding to the starting location. The second pollutant sensor 115 and the first pollutant sensor are identical pollutant sensors. The second pollutants and the first pollutants belong to identical pollutants and the second pollution parameter PO2 and the first pollution parameter PO1 belong to identical pollution parameters. The second image sensor 116 and the second thermal imaging camera 117 respectively capture a second visible light image V2 and a second thermal image T2 within the same range corresponding to the starting location. The second processor 111 obtains the second temperature range of the second thermal image T2. When the sensing location S is the starting location, the second processor 111 respectively uses the second pollution parameter PO2 and the second temperature range to replace the corresponding first pollution parameter PO1 and the corresponding first temperature range.

When the second pollution parameter PO2 is greater than a given value, the second processor 111 drives the portable display 112 to display that the sensing location S corresponding to the second pollution parameter PO2 greater than the given value is a polluted location. When the second visible light image V2 has a human face image and the second temperature range is greater than a given temperature for a given time period, the second processor 111 drives the portable display 112 to display the sensing position S corresponding to the second temperature greater than the given temperature is a polluted location. When the second visible light image V2 has a human face image and the second temperature range is greater than a given temperature, the second processor 111 drives the portable display 112 to display the human face image as a source of infection. When the second visible light image V2 has a human face image and the second pollution parameter PO2 is greater than a given value for a given time period, the second processor 111 drives the portable display 112 to display the human face image as a source of infection.

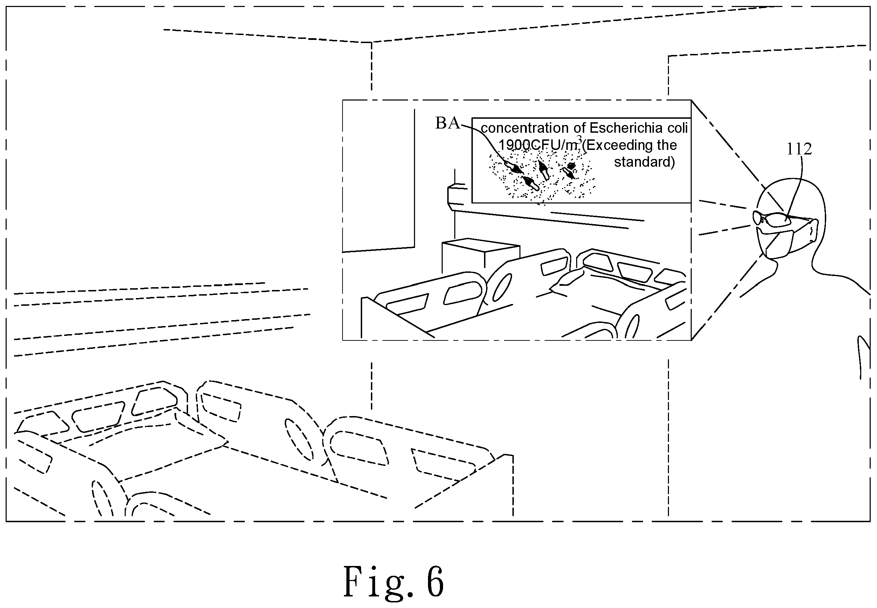

Fig.6 is a schematic diagram illustrating an image displayed by a portable display according to an embodiment of the present invention. Referring to Fig.6 and Fig.4, a portable display 112 may be a virtual reality (VR) display or an augmented reality (AR) display. The image displayed by the portable display 112 is shown in Fig.6. In Fig.6, the user wears the portable display 112 and the image shows that the concentration of Escherichia coli BA in the ward is 1900 colony forming units/cubic meter (CFU/m³).

Referring to Fig.1, Fig.2, and Fig.4, the system 1 for notifying environmental pollution status may further include at least one wireless base station 12 and a cloud server 13. The wireless base station 12 may be, but not limited to, a 3G base station, a 4G base station, a 5G base station, a Bluetooth (RTM) base station, or a WiFi base station. For convenience and clarity, the number of the wireless base station 12 is one. The wireless base station 12 is wirelessly connected to the first wireless communication modules 104 of the wireless environment sensing devices 10 and the second wireless communication module **110** of the portable wireless environmental pollution status notification device 11. The cloud server 13 is electrically connected to the wireless base station 12. The wireless environment sensing devices 10 transmit the pollution related information P and the sensing locations S corresponding thereto to the cloud server 13 through the wireless base station 12. The cloud server 13 transmits the pollution related information P and the sensing locations S corresponding thereto to the portable wireless environmental pollution status notification device 11 through the wireless base station 12.

Specifically, the first processor 105 uses the first pollution parameter PO1 as pollution related information P and transmits the sensing location S and the first pollution parameter PO1 to the cloud server 12 through the first wireless communication module 104 and the wireless base station 12.

When the first pollution parameter PO1 is greater than a given value, the first processor 105 notifies the second processor 111 of the portable wireless environmental pollution status notification device 11 through the first wireless communication module 104, the wireless base station 12, and the cloud server 13 that the sensing location S corresponding to the first pollution parameter PO1 greater than the given value is a polluted location. When the first visible light image V1 has a human face image and the first temperature range is greater than a given temperature for a given time period, the first processor 105 notifies the second processor 111 of the portable wireless environmental pollution status notification device 11 through the first wireless communication module 104, the wireless base station 12, and the cloud server 13 that the sensing location S corresponding to the first temperature range greater than the given temperature is a polluted location. When the first visible light image V1 has a human face image and the first temperature range is greater than a given temperature for a given time period, the first processor 105 transmits the human face image to the second processor 111 of the portable wireless environmental pollution status notification device 11 through the first wireless communication module 104, the wireless base station 12, and the cloud server 13. When the first visible light image V1 has a human face image and the first pollution parameter PO1 is greater than a given value for a given time period, the first processor 105 transmits the human face image to the second processor 111 of the portable wireless environmental pollution status notification device 11 through the first wireless communication module 104, the wireless base station 12, and the cloud server 13.

In addition, the second processor 111 may receive reference signal received power (RSRP), signal-to-interference-plus-noise ratios (SINRs), packet round-trip time (RTT), angles of arrival, angles of departure, and the location of the wireless base station 12 from the wireless base station 12 through the second wireless communication module 110 and calculate the location of the second processor 111 as a starting location based on the RSRP, the SINRs, the packet RTT, the angles of arrival, the angles of departure, and the location of the wireless base station 12.

According to the embodiments provided above, the system for notifying environmental pollution status positioning uses wireless environment sensing devices to implement environmental detection distribution in three-dimensional space, thereby providing a recommended path.

## Claims

1. A system (1) for notifying environmental pollution status comprising:
a plurality of wireless environment sensing devices (10) respectively arranged in different sensing locations of a physical environment (2) and configured to respectively store the sensing locations and respectively sense pollution related information corresponding to the different sensing locations to output the pollution related information and the sensing locations corresponding thereto; and
a portable wireless environmental pollution status notification device (11) wirelessly connected to the plurality of wireless environment sensing devices (10), located in the physical environment (2), and configured to receive the pollution related information and the sensing locations corresponding thereto and generate notification signals based on the pollution related information and the sensing locations corresponding thereto,
wherein each of the plurality of wireless environment sensing devices (10) includes:
at least one first pollutant sensor (100) configured to measure a first pollution parameter of first pollutants corresponding to the sensing location;
a first image sensor (101) and a first thermal imaging camera (102) configured to respectively capture a first visible light image and a first thermal image within the same range;
a storage device (103) configured to store the sensing location;
a first wireless communication module (104) wirelessly connected to the portable wireless environmental pollution status notification device (11); and
a first processor (105) electrically connected to the at least one first pollutant sensor (100), the first image sensor (101), the first thermal imaging camera (102), the storage device (103), and the first wireless communication module (104) and configured to extract a first temperature range of the first thermal image, use the first pollution parameter as the pollution related information, and transmit the sensing location and the first pollution parameter to the portable wireless environmental pollution status notification device (11) through the first wireless communication module (104),
wherein the notification signals include an image signal, a sound signal, and a vibration signal and the portable wireless environmental pollution status notification device (11) includes:
a second wireless communication module (110) wirelessly connected to the first wireless communication module (104);
a second processor (111) electrically connected to the second wireless communication module (110) and configured to receive the sensing location and the first pollution parameter through the second wireless communication module (110);
a portable display (112) electrically connected to the second processor (111), wherein the second processor (111) is configured to drive the portable display (112) to generate the image signal based on a given condition, the sensing location, and the first pollution parameter;
at least one portable sound player (113) electrically connected to the second processor (111), wherein the second processor (111) is configured to drive the at least one portable sound player (113) to generate the sound signal based on the sensing location and the first pollution parameter; and
a portable vibrator (114) electrically connected to the second processor (111), wherein the second processor (111) is configured to drive the portable vibrator (114) to generate the vibration signal based on the sensing location and the first pollution parameter,
being the system (1) **characterized in that** the given condition includes one of the different sensing locations as an ending location, the second processor is configured to receive reference signal received power (RSRP), signal-to-interference-plus-noise ratios (SINRs), packet round-trip time (RTT), angles of arrival, and angles of departure from the first wireless communication module (104) through the second wireless communication module (110) and calculate a starting location based on at least three of the different sensing locations and the RSRP, the SINRs, the packet RTT, the angles of arrival, and the angles of departure corresponding thereto, the second processor (111) is configured to plan candidate paths connected between the ending location and the starting location based on the different sensing locations, calculate risk values corresponding to the candidate paths, and select the candidate path corresponding to a highest one or a lowest one of the risk values as a recommended path, the risk $value = {\sum }_{i = 1}^{n} \frac{Pi}{Di}$, Di represents a distance between the starting location and a i-th the sensing location corresponding to the candidate path, Pi represents the first pollution parameter measured in a i-th the sensing location corresponding to the candidate path, n represents total number of the sensing locations corresponding to the candidate path, and the image signal shows the recommended path and an image corresponding to the sensing locations, the first pollutants, and the first pollution parameter.

2. The system (1) for notifying environmental pollution status according to claim 1, wherein the plurality of wireless environment sensing devices (10) are wirelessly connected to the portable wireless environmental pollution status notification device (11).

3. The system (1) for notifying environmental pollution status according to claim 1, wherein the first pollution parameter is a pollution concentration, a wind speed, a pollution range, or a flame burning intensity.

4. The system (1) for notifying environmental pollution status according to claim 1, wherein when the first pollution parameter is greater than a given value, the first processor (105) notifies the portable wireless environmental pollution status notification device (11) through the first wireless communication module (104) that the sensing location corresponding to the first pollution parameter greater than the given value is a polluted location.

5. The system (1) for notifying environmental pollution status according to claim 1, wherein when the first visible light image has a human face image and the first temperature range is greater than a given temperature for a given time period, the first processor (105) notifies the portable wireless environmental pollution status notification device (11) through the first wireless communication module (104) that the sensing location corresponding to the first temperature range greater than the given temperature is a polluted location.

6. The system (1) for notifying environmental pollution status according to claim 1, wherein when the first visible light image has a human face image and the first temperature range is greater than a given temperature for a given time period, the first processor (105) transmits the human face image to the portable wireless environmental pollution status notification device (11) through the first wireless communication module (104).

7. The system (1) for notifying environmental pollution status according to claim 1, wherein when the first visible light image has a human face image and the first pollution parameter is greater than a given value for a given time period, the first processor (105) transmits the human face image to the portable wireless environmental pollution status notification device (11) through the first wireless communication module (104).

8. The system (1) for notifying environmental pollution status according to claim 1, wherein the at least one first pollutant sensor (100) is an optical finger navigation (OFN) sensor.

9. The system (1) for notifying environmental pollution status according to claim 1, wherein the first pollutants include bacteria, mold, viruses, flame, smoke, liquefied gas, carbon monoxide, carbon dioxide, ozone, alkane gases, benzene gases, ketone gases, natural gas, coal gas, gasoline, chlorine, ammonia, flammable gases, harmful volatile matter, or airborne matter.

10. The system (1) for notifying environmental pollution status according to claim 1, wherein the at least one portable sound player (113) includes a plurality of portable sound players.

11. The system (1) for notifying environmental pollution status according to claim 1, wherein the portable wireless environmental pollution status notification device (11) further includes:
at least one second pollutant sensor (115) electrically connected to the second processor (11) and configured to measure a second pollution parameter of second pollutants corresponding to the starting location, wherein the second pollutants and the first pollutants belong to identical pollutants and the second pollution parameter and the first pollution parameter belong to identical pollution parameters; and
a second image sensor (116) and a second thermal imaging camera (117) electrically connected to the second processor (111) and configured to respectively capture a second visible light image and a second thermal image within the same range corresponding to the starting location, the second processor (111) is configured to obtain a second temperature range of the second thermal image, and when the sensing location is the starting location, the second processor (111) respectively uses the second pollution parameter and the second temperature range to replace a corresponding the first pollution parameter and a corresponding the first temperature range.

12. The system (1) for notifying environmental pollution status according to claim 11, wherein when the second pollution parameter is greater than a given value, the second processor (111) drives the portable display to display that the sensing location corresponding to the second pollution parameter greater than the given value is a polluted location.

13. The system (1) for notifying environmental pollution status according to claim 11, wherein when the second visible light image has a human face image and the second temperature range is greater than a given temperature for a given time period, the second processor (111) drives the portable display (112) to display the sensing position corresponding to the second temperature greater than the given temperature is a polluted location.

14. The system (1) for notifying environmental pollution status according to claim 11, wherein when the second visible light image has a human face image and the second temperature range is greater than a given temperature, the second processor (111) drives the portable display (112) to display the human face image as a source of infection.

15. The system (1) for notifying environmental pollution status according to claim 11, wherein when the second visible light image has a human face image and the second pollution parameter is greater than a given value for a given time period, the second processor (111) drives the portable display (112) to display the human face image as a source of infection.

16. A system for environmental pollution status notification, which comprises:
a plurality of wireless environment sensing devices (10) respectively arranged in different sensing locations of a physical environment (2) and configured to respectively store the sensing locations and respectively sense pollution related information corresponding to the different sensing locations to output the pollution related information and the sensing locations corresponding thereto;
a portable wireless environmental pollution status notification device (11) wirelessly connected to the plurality of wireless environment sensing devices (10), located in the physical environment (2), and configured to receive the pollution related information and the sensing locations corresponding thereto and generate notification signals based on the pollution related information and the sensing locations corresponding thereto;
at least one wireless base station (12) wirelessly connected to the plurality of wireless environment sensing devices (10) and the portable wireless environmental pollution status notification device (11); and
a cloud server (13) electrically connected to the at least one wireless base station (12), wherein the plurality of wireless environment sensing devices (10) are configured to transmit the pollution related information and the sensing locations corresponding thereto to the cloud server (13) through the at least one wireless base station (12), and the cloud server (13) is configured to transmit the pollution related information and the sensing locations corresponding thereto to the portable wireless environmental pollution status notification device (11) through the at least one wireless base station (12);
wherein each of the plurality of wireless environment sensing devices (10) includes:
at least one first pollutant sensor (100) configured to measure a first pollution parameter of first pollutants corresponding to the sensing location;
a first image sensor (101) and a first thermal imaging camera (102) configured to respectively capture a first visible light image and a first thermal image within the same range;
a storage device (103) configured to store the sensing location;
a first wireless communication module (104) wirelessly connected to the at least one wireless base station (12); and
a first processor (105) electrically connected to the at least one first pollutant sensor (100), the first image sensor (101), the first thermal imaging camera (102), the storage device (103), and the first wireless communication module (104) and configured to extract a first temperature range of the first thermal image, use the first pollution parameter as the pollution related information, and transmit the sensing location and the first pollution parameter to the cloud server (13) through the first wireless communication module (104) and the at least one wireless base station (12),
wherein the notification signals include an image signal, a sound signal, and a vibration signal and the portable wireless environmental pollution status notification device (11) includes:
a second wireless communication module (110) wirelessly connected to the at least one wireless base station (12);
a second processor (111) electrically connected to the second wireless communication module (110) and configured to receive the sensing location and the first pollution parameter through the second wireless communication module (110);
a portable display (112) electrically connected to the second processor (111), wherein the second processor (111) is configured to drive the portable display (112) to generate the image signal based on a given condition, the sensing location, and the first pollution parameter;
at least one portable sound player (113) electrically connected to the second processor (111), wherein the second processor (111) is configured to drive the at least one portable sound player (113) to generate the sound signal based on the sensing location and the first pollution parameter; and
a portable vibrator (114) electrically connected to the second processor (111), wherein the second processor (111) is configured to drive the portable vibrator (114) to generate the vibration signal based on the sensing location and the first pollution parameter,
being the system (1) **characterized in that** the given condition includes one of the different sensing locations as an ending location, the second processor (111) is configured to receive reference signal received power (RSRP), signal-to-interference-plus-noise ratios (SINRs), packet round-trip time (RTT), angles of arrival, angles of departure, and a location of the at least one wireless base station (12) from the at least one wireless base station (12) through the second wireless communication module (110) and calculate a starting location based on the RSRP, the SINRs, the packet RTT, the angles of arrival, the angles of departure, and the location of the at least one wireless base station (12), the second processor (111) is configured to plan candidate paths connected between the ending location and the starting location based on the different sensing locations, calculate risk values corresponding to the candidate paths, and select the candidate path corresponding to a highest one or a lowest one of the risk values as a recommended path, the risk $value = {\sum }_{i = 1}^{n} \frac{Pi}{Di}$, Di represents a distance between the starting location and a i-th the sensing location corresponding to the candidate path, Pi represents the first pollution parameter measured in a i-th the sensing location corresponding to the candidate path, n represents total number of the sensing locations corresponding to the candidate path, and the image signal shows the recommended path and an image corresponding to the sensing locations, the first pollutants, and the first pollution parameter.

17. The system (1) for notifying environmental pollution status according to claim 16, wherein the first pollution parameter is a pollution concentration, a wind speed, a pollution range, or a flame burning intensity.

18. The system (1) for notifying environmental pollution status according to claim 16, wherein when the first pollution parameter is greater than a given value, the first processor (105) notifies the portable wireless environmental pollution status notification device (11) through the first wireless communication module (104), the at least one wireless base station (12), and the cloud server (13) that the sensing location corresponding to the first pollution parameter greater than the given value is a polluted location.

19. The system (1) for notifying environmental pollution status according to claim 16, wherein when the first visible light image has a human face image and the first temperature range is greater than a given temperature for a given time period, the first processor (105) notifies the portable wireless environmental pollution status notification device (11) through the first wireless communication module (104), the at least one wireless base station (12), and the cloud server (13) that the sensing location corresponding to the first temperature range greater than the given temperature is a polluted location.

20. The system (1) for notifying environmental pollution status according to claim 16, wherein when the first visible light image has a human face image and the first temperature range is greater than a given temperature for a given time period, the first processor (105) transmits the human face image to the portable wireless environmental pollution status notification device (11) through the first wireless communication module (104), the at least one wireless base station (12), and the cloud server (13).

21. The system (1) for notifying environmental pollution status according to claim 16, wherein when the first visible light image has a human face image and the first pollution parameter is greater than a given value for a given time period, the first processor (105) transmits the human face image to the portable wireless environmental pollution status notification device (11) through the first wireless communication module (104), the at least one wireless base station (12), and the cloud server (13).

22. The system (1) for notifying environmental pollution status according to claim 16, wherein the at least one first pollutant sensor is an optical finger navigation (OFN) sensor.

23. The system (1) for notifying environmental pollution status according to claim 16, wherein the first pollutants include bacteria, mold, viruses, flame, smoke, liquefied gas, carbon monoxide, carbon dioxide, ozone, alkane gases, benzene gases, ketone gases, natural gas, coal gas, gasoline, chlorine, ammonia, flammable gases, harmful volatile matter, or airborne matter.

24. The system (1) for notifying environmental pollution status according to claim 16, wherein the at least one portable sound player (113) includes a plurality of portable sound players.

25. The system (1) for notifying environmental pollution status according to claim 16, wherein the portable wireless environmental pollution status notification device (11) further includes:
at least one second pollutant sensor (115) electrically connected to the second processor (111) and configured to measure a second pollution parameter of second pollutants corresponding to the starting location, wherein the second pollutants and the first pollutants belong to identical pollutants and the second pollution parameter and the first pollution parameter belong to identical pollution parameters; and
a second image sensor (116) and a second thermal imaging camera (117) electrically connected to the second processor (111) and configured to respectively capture a second visible light image and a second thermal image within the same range corresponding to the starting location, the second processor (111) is configured to obtain a second temperature range of the second thermal image, and when the sensing location is the starting location, the second processor (111) respectively uses the second pollution parameter and the second temperature range to replace a corresponding the first pollution parameter and a corresponding the first temperature range.

26. The system (1) for notifying environmental pollution status according to claim 25, wherein when the second pollution parameter is greater than a given value, the second processor (111) drives the portable display (112) to display that the sensing location corresponding to the second pollution parameter greater than the given value is a polluted location.

27. The system (1) for notifying environmental pollution status according to claim 25, wherein when the second visible light image has a human face image and the second temperature range is greater than a given temperature for a given time period, the second processor (111) drives the portable display (112) to display the sensing position corresponding to the second temperature greater than the given temperature is a polluted location.

28. The system (1) for notifying environmental pollution status according to claim 25, wherein when the second visible light image has a human face image and the second temperature range is greater than a given temperature, the second processor (111) drives the portable display (112) to display the human face image as a source of infection.

29. 0. The system (1) for notifying environmental pollution status according to claim 25, wherein when the second visible light image has a human face image and the second pollution parameter is greater than a given value for a given time period, the second processor (111) drives the portable display (112) to display the human face image as a source of infection.

## Patentansprüche

1. Ein System (1) zur Meldung des Umweltverschmutzungsstatus, umfassend:
eine Vielzahl von drahtlosen Umgebungssensoren (10), die jeweils an unterschiedlichen Messorten einer physischen Umgebung (2) angeordnet und so konfiguriert sind, dass sie jeweils die Messorte speichern und jeweils verschmutzungsbezogene Informationen erfassen, die den unterschiedlichen Messorten entsprechen, um die verschmutzungsbezogenen Informationen und die ihnen entsprechenden Messorte auszugeben; und
ein tragbares, drahtloses Gerät zur Meldung des Umweltverschmutzungsstatus, das drahtlos mit der Vielzahl von drahtlosen Umweltsensoren (10) verbunden ist, die sich in der physischen Umgebung (2) befinden, und so konfiguriert ist, dass es die Informationen zur Umweltverschmutzung und die diesen entsprechenden Messorte empfängt und Benachrichtigungssignale auf der Grundlage der Informationen zur Umweltverschmutzung und der diesen entsprechenden Messorte erzeugt,
wobei jedes der mehreren drahtlosen Umweltmessgeräte (10) umfasst:
mindestens einen ersten Schadstoffsensor (100), der so konfiguriert ist, dass er einen ersten Verschmutzungsparameter erster Schadstoffe entsprechend dem Messort misst;
einen ersten Bildsensor (101) und eine erste Wärmebildkamera (102), die so konfiguriert sind, dass sie jeweils ein erstes Bild im sichtbaren Licht und ein erstes Wärmebild innerhalb desselben Bereichs erfassen;
eine Speichervorrichtung (103), die so konfiguriert ist, dass sie den Erfassungsort speichert;
ein erstes drahtloses Kommunikationsmodul (104), das drahtlos mit dem tragbaren Gerät (11) zur drahtlosen Meldung des Umweltverschmutzungsstatus verbunden ist; und
einen ersten Prozessor (105), der elektrisch mit dem mindestens einen ersten Schadstoffsensor (100), dem ersten Bildsensor (101), der ersten Wärmebildkamera (102), der Speichervorrichtung (103) sowie mit dem ersten drahtlosen Kommunikationsmodul (104) elektrisch verbunden ist und so konfiguriert ist, dass er einen ersten Temperaturbereich des ersten Wärmebildes extrahiert, den ersten Schadstoffparameter als schadstoffbezogene Information verwendet und den Messort sowie den ersten Schadstoffparameter über das erste drahtlose Kommunikationsmodul (104) an das tragbare drahtlose Gerät zur Meldung des Umweltverschmutzungsstatus (11) überträgt,
wobei die Benachrichtigungssignale ein Bildsignal, ein Tonsignal und ein Vibrationssignal umfassen und das tragbare drahtlose Gerät zur Meldung des Umweltverschmutzungsstatus (11) umfasst:
ein zweites drahtloses Kommunikationsmodul (110), das drahtlos mit dem ersten drahtlosen Kommunikationsmodul (104) verbunden ist;
einen zweiten Prozessor (111), der elektrisch mit dem zweiten drahtlosen Kommunikationsmodul (110) verbunden und so konfiguriert ist, dass er den Messort und den ersten Verschmutzungsparameter über das zweite drahtlose Kommunikationsmodul (110) empfängt;
ein tragbares Display (112), das elektrisch mit dem zweiten Prozessor (111) verbunden ist, wobei der zweite Prozessor (111) so konfiguriert ist, dass er das tragbare Display (112) ansteuert, um das Bildsignal auf der Grundlage eines vorgegebenen Zustands, des Messorts und des ersten Verschmutzungsparameters zu erzeugen;
mindestens einen tragbaren Audioplayer (113), der elektrisch mit dem zweiten Prozessor (111) verbunden ist, wobei der zweite Prozessor (111) so konfiguriert ist, den mindestens einen tragbaren Audioplayer (113) so anzusteuern, dass er das Tonsignal auf der Grundlage des Erfassungsortes und des ersten Verschmutzungsparameters erzeugt; und
einen tragbaren Vibrator (114), der elektrisch mit dem zweiten Prozessor (111) verbunden ist, wobei der zweite Prozessor (111) so konfiguriert ist, dass er den tragbaren Vibrator (114) ansteuert, um das Vibrationssignal auf der Grundlage des Erfassungsortes und des ersten Verschmutzungsparameters zu erzeugen,
wobei das System (1) **dadurch gekennzeichnet ist, dass** die gegebene Bedingung einen der verschiedenen Erfassungsorte als Endort umfasst, der zweite Prozessor so konfiguriert ist, dass er die empfangene Referenzsignalleistung (RSRP), Signal-zu-Störsignalplus-Rausch-Verhältnisse (SINRs), die Paket-Round-Trip-Zeit (RTT), Ankunftswinkel und Abflugwinkel vom ersten drahtlosen Kommunikationsmodul (104) über das zweite drahtlose Kommunikationsmodul (110) zu empfangen und einen Startort auf der Grundlage von mindestens drei der verschiedenen Erfassungsorte und des RSRP, der SINRs, der Paket-RTT, der Ankunftswinkel und der entsprechenden Abflugwinkel zu berechnen; der zweite Prozessor (111) so konfiguriert ist, dass er auf der Grundlage der verschiedenen Erfassungsorte Kandidatenpfade plant, die den Endort mit dem Startort verbinden, Risikowerte entsprechend den Kandidatenpfaden berechnet und den Kandidatenpfad, der dem höchsten oder dem niedrigsten der Risikowerte entspricht, als empfohlenen Pfad auswählt, wobei der $Risikowert = {\sum }_{i = 1}^{n} \frac{Pi}{Di}$, wobei Di den Abstand zwischen dem Startort und dem i-ten Messort darstellt, der dem Kandidatenpfad entspricht, und Pi den ersten Verschmutzungsparameter darstellt, der am i-ten Messort entsprechend dem Kandidatenpfad gemessen wurde, wobei n die Gesamtzahl der Messstellen darstellt, die dem Kandidatenpfad entsprechen, und das Bildsignal den empfohlenen Pfad sowie ein Bild zeigt, das den Messstellen, den ersten Schadstoffen und dem ersten Schadstoffparameter entspricht.

2. System (1) zur Meldung des Umweltverschmutzungsstatus gemäß Anspruch 1, wobei die mehreren drahtlosen Umgebungserfassungsvorrichtungen (10) drahtlos mit der tragbaren drahtlosen Vorrichtung (11) zur Meldung des Umweltverschmutzungsstatus verbunden sind.

3. System (1) zur Meldung des Umweltverschmutzungsstatus gemäß Anspruch 1, wobei der erste Verschmutzungsparameter eine Schadstoffkonzentration, eine Windgeschwindigkeit, eine Verschmutzungsreichweite oder eine Flammenbrennintensität ist.

4. System (1) zur Meldung des Umweltverschmutzungsstatus gemäß Anspruch 1, wobei, wenn der erste Verschmutzungsparameter größer als ein vorgegebener Wert ist, der erste Prozessor (105) dem tragbaren drahtlosen Gerät zur Meldung des Umweltverschmutzungsstatus (11) über das erste drahtlose Kommunikationsmodul (104) mitteilt, dass der Erfassungsort, der dem ersten Verschmutzungsparameter entspricht, der größer als der vorgegebene Wert ist, ein verschmutzter Ort ist.

5. System (1) zur Meldung des Umweltverschmutzungsstatus gemäß Anspruch 1, wobei, wenn das erste Bild im sichtbaren Licht ein Bild eines menschlichen Gesichts enthält und der erste Temperaturbereich über einen vorgegebenen Zeitraum hinweg größer als eine vorgegebene Temperatur ist, der erste Prozessor (105) das tragbare drahtlose Gerät (11) zur Meldung des Umweltverschmutzungsstatus über das erste drahtlose Kommunikationsmodul (104) benachrichtigt, dass der Erfassungsort, der dem ersten Temperaturbereich entspricht, der größer ist als die vorgegebene Temperatur, ein verschmutzter Ort ist.

6. System (1) zur Meldung des Umweltverschmutzungsstatus gemäß Anspruch 1, wobei, wenn das erste Bild im sichtbaren Licht ein Bild eines menschlichen Gesichts enthält und der erste Temperaturbereich für einen vorgegebenen Zeitraum größer als eine vorgegebene Temperatur ist, der erste Prozessor (105) das Bild des menschlichen Gesichts über das erste drahtlose Kommunikationsmodul (104) an das tragbare drahtlose Gerät (11) zur Meldung des Umweltverschmutzungsstatus überträgt.

7. System (1) zur Meldung des Umweltverschmutzungsstatus gemäß Anspruch 1, wobei, wenn das erste Bild im sichtbaren Licht ein Bild eines menschlichen Gesichts enthält und der erste Verschmutzungsparameter über einen vorgegebenen Zeitraum hinweg größer als ein vorgegebener Wert ist, der erste Prozessor (105) das Bild des menschlichen Gesichts über das erste drahtlose Kommunikationsmodul (104) an das tragbare drahtlose Gerät (11) zur Meldung des Umweltverschmutzungsstatus überträgt.

8. System (1) zur Meldung des Umweltverschmutzungsstatus gemäß Anspruch 1, wobei der mindestens eine erste Schadstoffsensor (100) ein optischer Fingernavigationssensor (OFN-Sensor) ist.

9. System (1) zur Meldung des Umweltverschmutzungsstatus gemäß Anspruch 1, wobei die ersten Schadstoffe Bakterien, Schimmelpilze, Viren, Flammen, Rauch, Flüssiggas, Kohlenmonoxid, Kohlendioxid, Ozon, Alkan-Gase, Benzol-Gase, Keton-Gase, Erdgas, Kohlegas, Benzin, Chlor, Ammoniak, brennbare Gase, gesundheitsschädliche flüchtige Stoffe oder Schwebstoffe umfassen.

10. System (1) zur Meldung des Umweltverschmutzungsstatus gemäß Anspruch 1, wobei der mindestens eine tragbare Audioplayer (113) eine Vielzahl von tragbaren Audioplayern umfasst.

11. System (1) zur Meldung des Umweltverschmutzungsstatus gemäß Anspruch 1, wobei das tragbare, drahtlose Gerät (11) zur Meldung des Umweltverschmutzungsstatus ferner umfasst:
mindestens einen zweiten Schadstoffsensor (115), der elektrisch mit dem zweiten Prozessor (11) verbunden und so konfiguriert ist, dass er einen zweiten Schadstoffparameter von zweiten Schadstoffen misst, die dem Startort entsprechen, wobei die zweiten Schadstoffe und die ersten Schadstoffe zu identischen Schadstoffen gehören und der zweite Schadstoffparameter und der erste Schadstoffparameter zu identischen Schadstoffparametern gehören; und
einen zweiten Bildsensor (116) und eine zweite Wärmebildkamera (117), die elektrisch mit dem zweiten Prozessor (111) verbunden und so konfiguriert sind, dass sie jeweils ein zweites Bild im sichtbaren Licht und ein zweites Wärmebild innerhalb desselben Bereichs entsprechend dem Startort erfassen, wobei der zweite Prozessor (111) so konfiguriert ist, dass er einen zweiten Temperaturbereich des zweiten Wärmebilds ermittelt, und wenn der Erfassungsort der Startort ist, der zweite Prozessor (111) jeweils den zweiten Verschmutzungsparameter und den zweiten Temperaturbereich verwendet, um einen entsprechenden ersten Verschmutzungsparameter und einen entsprechenden ersten Temperaturbereich zu ersetzen.

12. System (1) zur Meldung des Umweltverschmutzungsstatus nach Anspruch 11, wobei, wenn der zweite Verschmutzungsparameter größer als ein vorgegebener Wert ist, der zweite Prozessor (111) das tragbare Display so ansteuert, dass angezeigt wird, dass der Erfassungsort, der dem zweiten Verschmutzungsparameter entspricht, der größer als der vorgegebene Wert ist, auf einen verschmutzten Ort hinweist.

13. System (1) zur Meldung des Umweltverschmutzungsstatus gemäß Anspruch 11, wobei, wenn das zweite Bild bei sichtbarem Licht ein Bild eines menschlichen Gesichts enthält und der zweite Temperaturbereich für einen vorgegebenen Zeitraum größer als eine vorgegebene Temperatur ist, der zweite Prozessor (111) das tragbare Display (112) so ansteuert, dass die Erfassungsposition, die der zweiten Temperatur entspricht, die größer als die vorgegebene Temperatur ist, als verschmutzter Ort angezeigt wird.

14. System (1) zur Meldung des Umweltverschmutzungsstatus gemäß Anspruch 11, wobei, wenn das zweite Bild im sichtbaren Licht ein menschliches Gesicht enthält und der zweite Temperaturbereich über einer vorgegebenen Temperatur liegt, der zweite Prozessor (111) das tragbare Display (112) so ansteuert, dass das menschliche Gesicht als Infektionsquelle angezeigt wird.

15. System (1) zur Meldung des Umweltverschmutzungsstatus gemäß Anspruch 11, wobei, wenn das zweite Bild im sichtbaren Licht ein menschliches Gesicht enthält und der zweite Verschmutzungsparameter über einen vorgegebenen Zeitraum hinweg größer als ein vorgegebener Wert ist, der zweite Prozessor (111) das tragbare Display (112) so ansteuert, dass das menschliche Gesichtsbild als Infektionsquelle angezeigt wird.

16. System zur Meldung des Umweltverschmutzungsstatus, das umfasst:
eine Vielzahl von drahtlosen Umgebungserfassungsgeräten (10), die jeweils an unterschiedlichen Erfassungsorten einer physikalischen Umgebung (2) angeordnet und so konfiguriert sind, dass sie jeweils die Erfassungsorte speichern und jeweils verschmutzungsbezogene Informationen entsprechend den unterschiedlichen Erfassungsorten erfassen, um die Verschmutzungsdaten und zugehörige Informationen und die diesen entsprechenden Messorte auszugeben;
ein tragbares, drahtloses Gerät (11) zur Meldung des Umweltverschmutzungsstatus, das drahtlos mit der Vielzahl von drahtlosen Umgebungserfassungsgeräten (10) verbunden ist, sich in der physischen Umgebung (2) befindet und so konfiguriert ist, dass es die Informationen zur Umweltverschmutzung und die entsprechenden Erfassungsorte empfängt und auf der Grundlage der Informationen zur Umweltverschmutzung und der entsprechenden Erfassungsorte Benachrichtigungssignale erzeugt;
mindestens eine drahtlose Basisstation (12), die drahtlos mit der Vielzahl von drahtlosen Umgebungserfassungsgeräten (10) und dem tragbaren drahtlosen Gerät zur Meldung des Umweltverschmutzungsstatus (11) verbunden ist; und
einen Cloud-Server (13), der elektrisch mit der mindestens einen drahtlosen Basisstation (12) verbunden ist, wobei die mehreren drahtlosen Umgebungserfassungsgeräte (10) so konfiguriert sind, dass sie die Informationen zur Umweltverschmutzung und die entsprechenden Erfassungsorte über die mindestens eine drahtlose Basisstation (12) an den Cloud-Server (13) übertragen, und der Cloud-Server (13) so konfiguriert ist, dass er die Informationen zur Umweltverschmutzung und die entsprechenden Messorte über die mindestens eine drahtlose Basisstation (12) an das tragbare drahtlose Gerät zur Meldung des Umweltverschmutzungsstatus (11) überträgt;
wobei jedes der mehreren drahtlosen Umgebungsmessgeräte (10) umfasst:
mindestens einen ersten Schadstoffsensor (100), der so konfiguriert ist, dass er einen ersten Schadstoffparameter von ersten Schadstoffen entsprechend dem Messort misst;
einen ersten Bildsensor (101) und eine erste Wärmebildkamera (102), die so konfiguriert sind, dass sie jeweils ein erstes Bild im sichtbaren Licht und ein erstes Wärmebild innerhalb desselben Bereichs erfassen;
eine Speichervorrichtung (103), die so konfiguriert ist, dass sie den Erfassungsort speichert;
ein erstes drahtloses Kommunikationsmodul (104), das drahtlos mit der mindestens einen drahtlosen Basisstation (12) verbunden ist; und
einen ersten Prozessor (105), der elektrisch mit dem mindestens einen ersten Schadstoffsensor (100), dem ersten Bildsensor (101), der ersten Wärmebildkamera (102), der Speichervorrichtung (103) sowie mit dem ersten drahtlosen Kommunikationsmodul (104) verbunden ist und so konfiguriert ist, einen ersten Temperaturbereich des ersten Wärmebildes zu extrahieren, den ersten Schadstoffparameter als schadstoffbezogene Information zu verwenden und den Messort sowie den ersten Schadstoffparameter über das erste drahtlose Kommunikationsmodul (104) und die mindestens eine drahtlose Basisstation (12) an den Cloud-Server (13) zu übertragen,
wobei die Benachrichtigungssignale ein Bildsignal, ein Tonsignal und ein Vibrationssignal umfassen, und das tragbare drahtlose Gerät (11) zur Meldung des Umweltverschmutzungsstatus umfasst:
ein zweites drahtloses Kommunikationsmodul (110), das drahtlos mit der mindestens einen drahtlosen Basisstation (12) verbunden ist;
einen zweiten Prozessor (111), der elektrisch mit dem zweiten drahtlosen Kommunikationsmodul (110) verbunden und so konfiguriert ist, dass er den Messort und den ersten Verschmutzungsparameter über das zweite drahtlose Kommunikationsmodul (110) empfängt;
ein tragbares Display (112), das elektrisch mit dem zweiten Prozessor (111) verbunden ist, wobei der zweite Prozessor (111) so konfiguriert ist, dass er das tragbare Display (112) ansteuert, um das Bildsignal auf der Grundlage eines vorgegebenen Zustands, des Messorts und des ersten Verschmutzungsparameters zu erzeugen;
mindestens ein tragbares Tonwiedergabegerät (113), das elektrisch mit dem zweiten Prozessor (111) verbunden ist, wobei der zweite Prozessor (111) so konfiguriert ist, dass er das mindestens eine tragbare Tonwiedergabegerät (113) ansteuert, um das Tonsignal auf der Grundlage des Erfassungsortes und des ersten Verschmutzungsparameters zu erzeugen; und
einen tragbaren Vibrator (114), der elektrisch mit dem zweiten Prozessor (111) verbunden ist, wobei der zweite Prozessor (111) so konfiguriert ist, dass er den tragbaren Vibrator (114) ansteuert, um das Vibrationssignal auf der Grundlage des Erfassungsortes und des ersten Verschmutzungsparameters zu erzeugen,
wobei das System (1) **dadurch gekennzeichnet ist, dass** die gegebene Bedingung einen der verschiedenen Erfassungsorte als Endort umfasst, der zweite Prozessor (111) so konfiguriert ist, dass er die empfangene Referenzsignalleistung (RSRP), Signal-zu-Störsignal-plus-Rausch-Verhältnisse (SINRs), die Paket-Hin- und Rücklaufzeit (RTT), Ankunftswinkel, Abflugwinkel und einen Standort der mindestens einen Funkbasisstation (12) von der mindestens einen Funkbasisstation (12) über das zweite Funkkommunikationsmodul (110) zu empfangen und einen Startort auf der Grundlage der RSRP, der SINRs, der Paket-RTT, der Ankunftswinkel, der Abflugwinkel und des Standorts der mindestens einen Funkbasisstation (12) zu berechnen, wobei der zweite Prozessor (111) so konfiguriert ist, dass er auf der Grundlage der verschiedenen Erfassungsstandorte mögliche Pfade zwischen dem Endstandort und dem Startstandort plant, den möglichen Pfaden entsprechende Risikowerte berechnet und den möglichen Pfad, der dem höchsten oder dem niedrigsten der Risikowerte entspricht, als empfohlenen Pfad auswählt, der $Risikowert = {\sum }_{i = 1}^{n} \frac{Pi}{Di} ,$, wobei Di den Abstand zwischen dem Startort und dem i-ten Messort darstellt, der dem Kandidatenweg entspricht, Pi den ersten Schadstoffparameter darstellt, der am i-ten Messort gemessen wurde, der dem Kandidatenweg entspricht, n die Gesamtzahl der Messorte darstellt, die dem Kandidatenweg entsprechen, und das Bildsignal den empfohlenen Weg sowie ein Bild zeigt, das den Messorten, den ersten Schadstoffen und dem ersten Schadstoffparameter entspricht.

17. System (1) zur Meldung des Umweltverschmutzungsstatus gemäß Anspruch 16, wobei der erste Verschmutzungsparameter eine Schadstoffkonzentration, eine Windgeschwindigkeit, eine Schadstoffreichweite oder eine Flammenbrennintensität ist.

18. System (1) zur Meldung des Umweltverschmutzungsstatus gemäß Anspruch 16, wobei, wenn der erste Verschmutzungsparameter größer als ein vorgegebener Wert ist, der erste Prozessor (105) das tragbare drahtlose Gerät (11) zur Meldung des Umweltverschmutzungsstatus über das erste drahtlose Kommunikationsmodul (104), die mindestens eine drahtlose Basisstation (12) und den Cloud-Server (13) darüber informiert, dass der Erfassungsort, der dem ersten Schadstoffparameter entspricht, der größer als der vorgegebene Wert ist, ein verschmutzter Ort ist.

19. System (1) zur Meldung des Umweltverschmutzungsstatus gemäß Anspruch 16, wobei, wenn das erste Bild im sichtbaren Licht ein Bild eines menschlichen Gesichts enthält und der erste Temperaturbereich für einen bestimmten Zeitraum größer als eine vorgegebene Temperatur ist, der erste Prozessor (105) das tragbare drahtlose Gerät zur Meldung des Umweltverschmutzungsstatus (11) über das erste drahtlose Kommunikationsmodul (104), die mindestens eine drahtlose Basisstation (12) und den Cloud-Server (13) darüber benachrichtigt, dass der Erfassungsort, der dem ersten Temperaturbereich über der vorgegebenen Temperatur entspricht, ein verschmutzter Ort ist.

20. System (1) zur Meldung des Umweltverschmutzungsstatus gemäß Anspruch 16, wobei, wenn das erste Bild im sichtbaren Licht ein menschliches Gesicht enthält und der erste Temperaturbereich über einen bestimmten Zeitraum hinweg größer als eine vorgegebene Temperatur ist, der erste Prozessor (105) das Bild des menschlichen Gesichts über das erste drahtlose Kommunikationsmodul (104), die mindestens eine drahtlose Basisstation (12) und den Cloud-Server (13) an das tragbare drahtlose Gerät zur Meldung des Umweltverschmutzungsstatus übermittelt.

21. System (1) zur Meldung des Umweltverschmutzungsstatus gemäß Anspruch 16, wobei, wenn das erste Bild im sichtbaren Licht ein Bild eines menschlichen Gesichts enthält und der erste Verschmutzungsparameter über einen bestimmten Zeitraum hinweg größer als ein vorgegebener Wert ist, der erste Prozessor (105) das Bild des menschlichen Gesichts über das erste drahtlose Kommunikationsmodul (104), die mindestens eine drahtlose Basisstation (12) und den Cloud-Server (13) an das tragbare drahtlose Gerät zur Meldung des Umweltverschmutzungsstatus übermittelt.

22. System (1) zur Meldung des Umweltverschmutzungsstatus gemäß Anspruch 16, wobei der mindestens eine erste Schadstoffsensor ein optischer Finger-Navigationssensor (OFN) ist.

23. System (1) zur Meldung des Umweltverschmutzungsstatus gemäß Anspruch 16, wobei die ersten Schadstoffe Bakterien, Schimmelpilze, Viren, Flammen, Rauch, Flüssiggas, Kohlenmonoxid, Kohlendioxid, Ozon, Alkan-Gase, Benzol-Gase, Keton-Gase, Erdgas, Kohlegas, Benzin, Chlor, Ammoniak, brennbare Gase, gesundheitsschädliche flüchtige Stoffe oder Schwebstoffe umfassen.

24. System (1) zur Meldung des Umweltverschmutzungsstatus gemäß Anspruch 16, wobei der mindestens eine tragbare Audioplayer (113) eine Vielzahl von tragbaren Audioplayern umfasst.

25. System (1) zur Meldung des Umweltverschmutzungsstatus gemäß Anspruch 16, wobei das tragbare, drahtlose Gerät (11) zur Meldung des Umweltverschmutzungsstatus ferner umfasst:
mindestens einen zweiten Schadstoffsensor (115), der elektrisch mit dem zweiten Prozessor (111) verbunden und so konfiguriert ist, dass er einen zweiten Schadstoffparameter von zweiten Schadstoffen misst, die dem Startort entsprechen, wobei die zweiten Schadstoffe und die ersten Schadstoffe zu identischen Schadstoffen gehören und der zweite Schadstoffparameter und der erste Schadstoffparameter zu identischen Schadstoffparametern gehören; und
einen zweiten Bildsensor (116) und eine zweite Wärmebildkamera (117), die elektrisch mit dem zweiten Prozessor (111) verbunden und so konfiguriert sind, dass sie jeweils ein zweites Bild im sichtbaren Licht und ein zweites Wärmebild innerhalb desselben Bereichs entsprechend dem Startort erfassen, wobei der zweite Prozessor (111) so konfiguriert ist, dass er einen zweiten Temperaturbereich des zweiten Wärmebilds ermittelt, und wenn der Erfassungsort der Startort ist, der zweite Prozessor (111) jeweils den zweiten Verschmutzungsparameter und den zweiten Temperaturbereich verwendet, um einen entsprechenden ersten Verschmutzungsparameter und einen entsprechenden ersten Temperaturbereich zu ersetzen.

26. System (1) zur Meldung des Umweltverschmutzungsstatus gemäß Anspruch 25,
wobei, wenn der zweite Verschmutzungsparameter größer als ein vorgegebener Wert ist, der zweite Prozessor (111) die tragbare Anzeige (112) so ansteuert, dass der dem zweiten Verschmutzungsparameter, der größer als der vorgegebene Wert ist, entsprechende Erfassungsort als ein verschmutzter Ort angezeigt wird.

27. System (1) zur Meldung des Umweltverschmutzungsstatus gemäß Anspruch 25, wobei, wenn das zweite Bild im sichtbaren Licht ein Bild eines menschlichen Gesichts enthält und der zweite Temperaturbereich über einen bestimmten Zeitraum hinweg größer ist als eine vorgegebene Temperatur, der zweite Prozessor (111) das tragbare Display (112) so ansteuert, dass die Erfassungsposition, die der zweiten Temperatur entspricht, die größer ist als die vorgegebene Temperatur, als verschmutzter Ort angezeigt wird.

28. System (1) zur Meldung des Umweltverschmutzungsstatus gemäß Anspruch 25, wobei, wenn das zweite Bild im sichtbaren Licht ein Bild eines menschlichen Gesichts enthält und der zweite Temperaturbereich über einer vorgegebenen Temperatur liegt, der zweite Prozessor (111) das tragbare Display (112) so ansteuert, dass das Bild des menschlichen Gesichts als Infektionsquelle angezeigt wird.

29. System (1) zur Meldung des Umweltverschmutzungsstatus gemäß Anspruch 25, wobei, wenn das zweite Bild im sichtbaren Licht ein Bild eines menschlichen Gesichts enthält und der zweite Verschmutzungsparameter größer ist als ein vorgegebener Wert für einen vorgegebenen Zeitraum, der zweite Prozessor (111) das tragbare Display (112) so ansteuert, dass das Bild des menschlichen Gesichts als Infektionsquelle angezeigt wird.

## Revendications

1. Système (1) de notification d'un statut de pollution environnementale comprenant :
une pluralité de dispositifs de détection environnementale sans fil (10) agencés respectivement à différents emplacements de détection d'un environnement physique (2) et configurés pour stocker respectivement les emplacements de détection et détecter respectivement des informations relatives à la pollution correspondant aux différents emplacements de détection afin de délivrer les informations relatives à la pollution et les emplacements de détection y correspondant ; et
un dispositif portable sans fil de notification de statut de pollution environnementale (11) connecté sans fil à la pluralité de dispositifs de détection environnementale sans fil (10), situé dans l'environnement physique (2), et configuré pour recevoir les informations relatives à la pollution et les emplacements de détection y correspondant et générer des signaux de notification sur la base des informations relatives à la pollution et des emplacements de détection y correspondant,
dans lequel chacun de la pluralité de dispositifs de détection environnementale sans fil (10) comporte :
au moins un premier capteur de polluants (100) configuré pour mesurer un premier paramètre de pollution de premiers polluants correspondant à l'emplacement de détection ;
un premier capteur d'image (101) et une première caméra d'imagerie thermique (102) configurés pour capturer respectivement une première image de lumière visible et une première image thermique dans la même zone ;
un dispositif de stockage (103) configuré pour stocker l'emplacement de détection ;
un premier module de communication sans fil (104) connecté sans fil au dispositif portable sans fil de notification de statut de pollution environnementale (11) ; et
un premier processeur (105) connecté électriquement à l'au moins un premier capteur de polluants (100), au premier capteur d'image (101), à la première caméra d'imagerie thermique (102), au dispositif de stockage (103) et au premier module de communication sans fil (104), et configuré pour extraire une première plage de températures de la première image thermique, utiliser le premier paramètre de pollution comme informations relatives à la pollution, et transmettre l'emplacement de détection et le premier paramètre de pollution au dispositif portable sans fil de notification de statut de pollution environnementale (11) via le premier module de communication sans fil (104),
dans lequel les signaux de notification comportent un signal d'image, un signal sonore et un signal de vibration, et le dispositif portable sans fil de notification de statut de pollution environnementale (11) comporte :
un deuxième module de communication sans fil (110) connecté sans fil au premier module de communication sans fil (104) ;
un deuxième processeur (111) connecté électriquement au deuxième module de communication sans fil (110) et configuré pour recevoir l'emplacement de détection et le premier paramètre de pollution via le deuxième module de communication sans fil (110) ;
un affichage portable (112) connecté électriquement au deuxième processeur (111), dans lequel le deuxième processeur (111) est configuré pour commander l'affichage portable (112) pour générer le signal d'image sur la base d'une condition donnée, de l'emplacement de détection et du premier paramètre de pollution ;
au moins un lecteur audio portable (113) connecté électriquement au deuxième processeur (111), dans lequel le deuxième processeur (111) est configuré pour commander l'au moins un lecteur audio portable (113) pour générer le signal sonore sur la base de l'emplacement de détection et du premier paramètre de pollution ; et
un vibrateur portable (114) connecté électriquement au deuxième processeur (111), dans lequel le deuxième processeur (111) est configuré pour commander le vibrateur portable (114) pour générer le signal de vibration sur la base de l'emplacement de détection et du premier paramètre de pollution,
le système (1) étant **caractérisé en ce que** la condition donnée comporte l'un des différents emplacements de détection en tant qu'emplacement de fin, le deuxième processeur est configuré pour recevoir du premier module de communication sans fil (104) une puissance du signal de référence reçu (RSRP), des rapports signal sur interférence plus bruit (SINR), des temps aller-retour (RTT) des paquets, des angles d'arrivée et des angles de départ via le deuxième module de communication sans fil (110), et pour calculer un emplacement de départ sur la base d'au moins trois des différents emplacements de détection et de la RSRP, des SINR, du RTT des paquets, des angles d'arrivée et des angles de départ y correspondant, le deuxième processeur (111) est configuré pour planifier des chemins candidats connectés entre l'emplacement de fin et l'emplacement de départ sur la base des différents emplacements de détection, calculer des valeurs de risque correspondant aux chemins candidats, et sélectionner le chemin candidat correspondant à une valeur la plus élevée ou la plus faible des valeurs de risque en tant que chemin recommandé, la valeur de $risque = {\sum }_{i = 1}^{n} \frac{Pi}{Di}$, *Di* représente une distance entre l'emplacement de départ et un i^{ème} emplacement de détection correspondant au chemin candidat, *Pi* représente le premier paramètre de pollution mesuré dans un i^{ème} emplacement de détection correspondant au chemin candidat, *n* représente le nombre total des emplacements de détection correspondant au chemin candidat, et le signal d'image montre le chemin recommandé et une image correspondant aux emplacements de détection, aux premiers polluants et au premier paramètre de pollution.

2. Système (1) de notification d'un statut de pollution environnementale selon la revendication 1, dans lequel la pluralité de dispositifs de détection environnementale sans fil (10) sont connectés sans fil au dispositif portable sans fil de notification de statut de pollution environnementale (11).

3. Système (1) de notification d'un statut de pollution environnementale selon la revendication 1, dans lequel le premier paramètre de pollution est une concentration de pollution, une vitesse du vent, une plage de pollution ou une intensité de combustion de flamme.

4. Système (1) de notification d'un statut de pollution environnementale selon la revendication 1, dans lequel, lorsque le premier paramètre de pollution est supérieur à une valeur donnée, le premier processeur (105) signale au dispositif portable sans fil de notification de statut de pollution environnementale (11) via le premier module de communication sans fil (104) que l'emplacement de détection correspondant au premier paramètre de pollution supérieur à la valeur donnée est un emplacement pollué.

5. Système (1) de notification d'un statut de pollution environnementale selon la revendication 1, dans lequel, lorsque la première image de lumière visible comporte une image de visage humain et que la première plage de températures est supérieure à une température donnée pendant une période de temps donnée, le premier processeur (105) signale au dispositif portable sans fil de notification de statut de pollution environnementale (11) via le premier module de communication sans fil (104) que l'emplacement de détection correspondant à la première plage de températures supérieure à la température donnée est un emplacement pollué.

6. Système (1) de notification d'un statut de pollution environnementale selon la revendication 1, dans lequel, lorsque la première image de lumière visible comporte une image de visage humain et que la première plage de températures est supérieure à une température donnée pendant une période de temps donnée, le premier processeur (105) transmet l'image de visage humain au dispositif portable sans fil de notification de statut de pollution environnementale (11) via le premier module de communication sans fil (104).

7. Système (1) de notification d'un statut de pollution environnementale selon la revendication 1, dans lequel, lorsque la première image de lumière visible comporte une image de visage humain et que le premier paramètre de pollution est supérieur à une valeur donnée pendant une période de temps donnée, le premier processeur (105) transmet l'image de visage humain au dispositif portable sans fil de notification de statut de pollution environnementale (11) via le premier module de communication sans fil (104).

8. Système (1) de notification d'un statut de pollution environnementale selon la revendication 1, dans lequel l'au moins un premier capteur de polluants (100) est un capteur de navigation optique au doigt (OFN).

9. Système (1) de notification d'un statut de pollution environnementale selon la revendication 1, dans lequel les premiers polluants comportent des bactéries, des moisissures, des virus, des flammes, de la fumée, du gaz liquéfié, du monoxyde de carbone, du dioxyde de carbone, de l'ozone, des gaz alcanes, des gaz benzéniques, des gaz cétoniques, du gaz naturel, du gaz de houille, de l'essence, du chlore, de l'ammoniac, des gaz inflammables, des matières volatiles nocives ou des matières en suspension dans l'air.

10. Système (1) de notification d'un statut de pollution environnementale selon la revendication 1, dans lequel l'au moins un lecteur audio portable (113) comporte une pluralité de lecteurs audio portables.

11. Système (1) de notification d'un statut de pollution environnementale selon la revendication 1, dans lequel le dispositif portable sans fil de notification de statut de pollution environnementale (11) comporte en outre :
au moins un deuxième capteur de polluants (115) connecté électriquement au deuxième processeur (11) et configuré pour mesurer un deuxième paramètre de pollution de deuxièmes polluants correspondant à l'emplacement de départ, dans lequel les deuxièmes polluants et les premiers polluants font partie de polluants identiques, et le deuxième paramètre de pollution et le premier paramètre de pollution font partie de paramètres de pollution identiques ; et
un deuxième capteur d'image (116) et une deuxième caméra d'imagerie thermique (117) connectés électriquement au deuxième processeur (111) et configurés pour capturer respectivement une deuxième image de lumière visible et une deuxième image thermique dans la même zone correspondant à l'emplacement de départ, le deuxième processeur (111) est configuré pour obtenir une deuxième plage de températures de la deuxième image thermique, et lorsque l'emplacement de détection est l'emplacement de départ, le deuxième processeur (111) utilise respectivement le deuxième paramètre de pollution et la deuxième plage de températures pour remplacer un premier paramètre de pollution correspondant et une première plage de températures correspondante.

12. Système (1) de notification d'un statut de pollution environnementale selon la revendication 11, dans lequel, lorsque le deuxième paramètre de pollution est supérieur à une valeur donnée, le deuxième processeur (111) commande l'affichage portable pour afficher l'emplacement de détection correspondant au deuxième paramètre de pollution supérieur à la valeur donnée comme étant un emplacement pollué.

13. Système (1) de notification d'un statut de pollution environnementale selon la revendication 11, dans lequel, lorsque la deuxième image de lumière visible comporte une image de visage humain et que la deuxième plage de températures est supérieure à une température donnée pendant une période de temps donnée, le deuxième processeur (111) commande l'affichage portable (112) pour afficher la position de détection correspondant à la deuxième température supérieure à la température donnée comme étant un emplacement pollué.

14. Système (1) de notification d'un statut de pollution environnementale selon la revendication 11, dans lequel, lorsque la deuxième image de lumière visible comporte une image de visage humain et que la deuxième plage de températures est supérieure à une température donnée, le deuxième processeur (111) commande l'affichage portable (112) pour afficher l'image de visage humain comme étant une source d'infection.

15. Système (1) de notification d'un statut de pollution environnementale selon la revendication 11, dans lequel, lorsque la deuxième image de lumière visible comporte une image de visage humain et que le deuxième paramètre de pollution est supérieur à une valeur donnée pendant une période de temps donnée, le deuxième processeur (111) commande l'affichage portable (112) pour afficher l'image de visage humain comme étant une source d'infection.

16. Système de notification de statut de pollution environnementale qui comprend :
une pluralité de dispositifs de détection environnementale sans fil (10) agencés respectivement à différents emplacements de détection d'un environnement physique (2) et configurés pour stocker respectivement les emplacements de détection et détecter respectivement des informations relatives à la pollution correspondant aux différents emplacements de détection afin de délivrer les informations relatives à la pollution et les emplacements de détection y correspondant ;
un dispositif portable sans fil de notification de statut de pollution environnementale (11) connecté sans fil à la pluralité de dispositifs de détection environnementale sans fil (10), situé dans l'environnement physique (2), et configuré pour recevoir les informations relatives à la pollution et les emplacements de détection y correspondant et générer des signaux de notification sur la base des informations relatives à la pollution et des emplacements de détection y correspondant ;
au moins une station de base sans fil (12) connectée sans fil à la pluralité de dispositifs de détection environnementale sans fil (10) et au dispositif portable sans fil de notification de statut de pollution environnementale (11) ; et
un serveur en nuage (13) connecté électriquement à l'au moins une station de base sans fil (12), dans lequel la pluralité de dispositifs de détection environnementale sans fil (10) sont configurés pour transmettre les informations relatives à la pollution et les emplacements de détection y correspondant au serveur en nuage (13) via l'au moins une station de base sans fil (12), et le serveur en nuage (13) est configuré pour transmettre les informations relatives à la pollution et les emplacements de détection y correspondant au dispositif portable sans fil de notification de statut de pollution environnementale (11) via l'au moins une station de base sans fil (12) ;
dans lequel chacun de la pluralité de dispositifs de détection environnementale sans fil (10) comporte :
au moins un premier capteur de polluants (100) configuré pour mesurer un premier paramètre de pollution de premiers polluants correspondant à l'emplacement de détection ;
un premier capteur d'image (101) et une première caméra d'imagerie thermique (102) configurés pour capturer respectivement une première image de lumière visible et une première image thermique dans la même zone ;
un dispositif de stockage (103) configuré pour stocker l'emplacement de détection ;
un premier module de communication sans fil (104) connecté sans fil à l'au moins une station de base sans fil (12) ; et
un premier processeur (105) connecté électriquement à l'au moins un premier capteur de polluants (100), au premier capteur d'image (101), à la première caméra d'imagerie thermique (102), au dispositif de stockage (103) et au premier module de communication sans fil (104), et configuré pour extraire une première plage de températures de la première image thermique, utiliser le premier paramètre de pollution comme informations relatives à la pollution, et transmettre l'emplacement de détection et le premier paramètre de pollution au serveur en nuage (13) via le premier module de communication sans fil (104) et l'au moins une station de base sans fil (12),
dans lequel les signaux de notification comportent un signal d'image, un signal sonore et un signal de vibration, et le dispositif portable sans fil de notification de statut de pollution environnementale (11) comporte :
un deuxième module de communication sans fil (110) connecté sans fil à l'au moins une station de base sans fil (12) ;
un deuxième processeur (111) connecté électriquement au deuxième module de communication sans fil (110) et configuré pour recevoir l'emplacement de détection et le premier paramètre de pollution via le deuxième module de communication sans fil (110) ;
un affichage portable (112) connecté électriquement au deuxième processeur (111), dans lequel le deuxième processeur (111) est configuré pour commander l'affichage portable (112) pour générer le signal d'image sur la base d'une condition donnée, de l'emplacement de détection et du premier paramètre de pollution ;
au moins un lecteur audio portable (113) connecté électriquement au deuxième processeur (111), dans lequel le deuxième processeur (111) est configuré pour commander l'au moins un lecteur audio portable (113) pour générer le signal sonore sur la base de l'emplacement de détection et du premier paramètre de pollution ; et
un vibrateur portable (114) connecté électriquement au deuxième processeur (111), dans lequel le deuxième processeur (111) est configuré pour commander le vibrateur portable (114) pour générer le signal de vibration sur la base de l'emplacement de détection et du premier paramètre de pollution,
le système (1) étant **caractérisé en ce que** la condition donnée comporte l'un des différents emplacements de détection en tant qu'emplacement de fin, le deuxième processeur (111) est configuré pour recevoir de l'au moins une station de base sans fil (12) une puissance du signal de référence reçu (RSRP), des rapports signal sur interférence plus bruit (SINR), des temps aller-retour (RTT) des paquets , des angles d'arrivée, des angles de départ et un emplacement de l'au moins une station de base sans fil (12) via le deuxième module de communication sans fil (110), et pour calculer un emplacement de départ sur la base de la RSRP, des SINR, du RTT des paquets, des angles d'arrivée, des angles de départ et de l'emplacement de l'au moins une station de base sans fil (12), le deuxième processeur (111) est configuré pour planifier des chemins candidats connectés entre l'emplacement de fin et l'emplacement de départ sur la base des différents emplacements de détection, calculer des valeurs de risque correspondant aux chemins candidats, et sélectionner le chemin candidat correspondant à une valeur la plus élevée ou la plus faible des valeurs de risque en tant que chemin recommandé, la valeur de $risque = {\sum }_{i = 1}^{n} \frac{Pi}{Di}$, *Di* représente une distance entre l'emplacement de départ et un i^{ème} emplacement de détection correspondant au chemin candidat, *Pi* représente le premier paramètre de pollution mesuré dans un i^{ème} emplacement de détection correspondant au chemin candidat, *n* représente le nombre total des emplacements de détection correspondant au chemin candidat, et le signal d'image montre le chemin recommandé et une image correspondant aux emplacements de détection, aux premiers polluants et au premier paramètre de pollution.

17. Système (1) de notification d'un statut de pollution environnementale selon la revendication 16, dans lequel le premier paramètre de pollution est une concentration de pollution, une vitesse du vent, une plage de pollution ou une intensité de combustion de flamme.

18. Système (1) de notification d'un statut de pollution environnementale selon la revendication 16, dans lequel, lorsque le premier paramètre de pollution est supérieur à une valeur donnée, le premier processeur (105) signale au dispositif portable sans fil de notification de statut de pollution environnementale (11) via le premier module de communication sans fil (104), l'au moins une station de base sans fil (12) et le serveur en nuage (13) que l'emplacement de détection correspondant au premier paramètre de pollution supérieur à la valeur donnée est un emplacement pollué.

19. Système (1) de notification d'un statut de pollution environnementale selon la revendication 16, dans lequel, lorsque la première image de lumière visible comporte une image de visage humain et que la première plage de températures est supérieure à une température donnée pendant une période de temps donnée, le premier processeur (105) signale au dispositif portable sans fil de notification de statut de pollution environnementale (11) via le premier module de communication sans fil (104), l'au moins une station de base sans fil (12) et le serveur en nuage (13) que l'emplacement de détection correspondant à la première plage de températures supérieure à la température donnée est un emplacement pollué.

20. Système (1) de notification d'un statut de pollution environnementale selon la revendication 16, dans lequel, lorsque la première image de lumière visible comporte une image de visage humain et que la première plage de températures est supérieure à une température donnée pendant une période de temps donnée, le premier processeur (105) transmet l'image de visage humain au dispositif portable sans fil de notification de statut de pollution environnementale (11) via le premier module de communication sans fil (104), l'au moins une station de base sans fil (12) et le serveur en nuage (13).

21. Système (1) de notification d'un statut de pollution environnementale selon la revendication 16, dans lequel, lorsque la première image de lumière visible comporte une image de visage humain et que le premier paramètre de pollution est supérieur à une valeur donnée pendant une période de temps donnée, le premier processeur (105) transmet l'image de visage humain au dispositif portable sans fil de notification de statut de pollution environnementale (11) via le premier module de communication sans fil (104), l'au moins une station de base sans fil (12) et le serveur en nuage (13).

22. Système (1) de notification d'un statut de pollution environnementale selon la revendication 16, dans lequel l'au moins un premier capteur de polluants est un capteur de navigation optique au doigt (OFN).

23. Système (1) de notification d'un statut de pollution environnementale selon la revendication 16, dans lequel les premiers polluants comportent des bactéries, des moisissures, des virus, des flammes, de la fumée, du gaz liquéfié, du monoxyde de carbone, du dioxyde de carbone, de l'ozone, des gaz alcanes, des gaz benzéniques, des gaz cétoniques, du gaz naturel, du gaz de houille, de l'essence, du chlore, de l'ammoniac, des gaz inflammables, des matières volatiles nocives ou des matières en suspension dans l'air.

24. Système (1) de notification d'un statut de pollution environnementale selon la revendication 16, dans lequel l'au moins un lecteur audio portable (113) comporte une pluralité de lecteurs audio portables.

25. Système (1) de notification d'un statut de pollution environnementale selon la revendication 16, dans lequel le dispositif portable sans fil de notification de statut de pollution environnementale (11) comporte en outre :
au moins un deuxième capteur de polluants (115) connecté électriquement au deuxième processeur (111) et configuré pour mesurer un deuxième paramètre de pollution de deuxièmes polluants correspondant à l'emplacement de départ, dans lequel les deuxièmes polluants et les premiers polluants font partie de polluants identiques, et le deuxième paramètre de pollution et le premier paramètre de pollution font partie de paramètres de pollution identiques ; et
un deuxième capteur d'image (116) et une deuxième caméra d'imagerie thermique (117) connectés électriquement au deuxième processeur (111) et configurés pour capturer respectivement une deuxième image de lumière visible et une deuxième image thermique dans la même zone correspondant à l'emplacement de départ, le deuxième processeur (111) est configuré pour obtenir une deuxième plage de températures de la deuxième image thermique, et lorsque l'emplacement de détection est l'emplacement de départ, le deuxième processeur (111) utilise respectivement le deuxième paramètre de pollution et la deuxième plage de températures pour remplacer un premier paramètre de pollution correspondant et une première plage de températures correspondante.

26. Système (1) de notification d'un statut de pollution environnementale selon la revendication 25, dans lequel, lorsque le deuxième paramètre de pollution est supérieur à une valeur donnée, le deuxième processeur (111) commande l'affichage portable (112) pour afficher l'emplacement de détection correspondant au deuxième paramètre de pollution supérieur à la valeur donnée comme étant un emplacement pollué.

27. Système (1) de notification d'un statut de pollution environnementale selon la revendication 25, dans lequel, lorsque la deuxième image de lumière visible comporte une image de visage humain et que la deuxième plage de températures est supérieure à une température donnée pendant une période de temps donnée, le deuxième processeur (111) commande l'affichage portable (112) pour afficher la position de détection correspondant à la deuxième température supérieure à la température donnée comme étant un emplacement pollué.

28. Système (1) de notification d'un statut de pollution environnementale selon la revendication 25, dans lequel, lorsque la deuxième image de lumière visible comporte une image de visage humain et que la deuxième plage de températures est supérieure à une température donnée, le deuxième processeur (111) commande l'affichage portable (112) pour afficher l'image de visage humain comme étant une source d'infection.

29. Système (1) de notification d'un statut de pollution environnementale selon la revendication 25, dans lequel, lorsque la deuxième image de lumière visible comporte une image de visage humain et que le deuxième paramètre de pollution est supérieur à une valeur donnée pendant une période de temps donnée, le deuxième processeur (111) commande l'affichage portable (112) pour afficher l'image de visage humain comme étant une source d'infection.
